# EUROPEAN PATENT APPLICATION

(11) **EP 1 742 059 A1**
(43) Date of publication of application: **10.01.2007**
(21) Application number: 04029171.8
(22) Date of filing: 09.12.2004
(51) Int. Cl.: G01N 33/574, C07K 14/575, C07K 16/26, C12N 15/00, C12N 5/00, A61P 35/00

(54) **Use of PTA peptides for stratification of individuals having cancer**

(71) Applicant: DIGILAB BioVisioN GmbH, 30625 Hannover (DE)
(72) Inventor: Tammen, Herald, Dr., 30449 Hannover (DE); Pich, Andreas, Dr., 31303 Burgdorf (DE); Traub, Frank, Dr., 30161 Hannover (DE); Hess, Rüdiger, Dr., 30629Hannover (DE); Lamping, Norbert, Dr., 30172 Hannover (DE); Schorn, Karl, Dr., 30179 Hannover (DE); Kreipe, Hans, Prof. Dr., 30982 Pattensen (DE)
(74) Representative: Kröncke, Rolf

(57) **Abstract**

Several types of cancer cells are treated with specialized therapies, as they have specific properties such as steroid-dependant, especially estradiol-dependant growth. Breast cancer and colon cancer cells often depend to some extend on estradiol-mediated growth signals. Therefore, especially breast cancer patients are routinely tested for the presence of estradiol receptors by immune histology. If the estradiol receptor is present usually an anti-estradiol therapy is used to eradicate the tumour and to prevent rezidives. However to a certain extend this therapy does not work and it would be an advantage to know this as early as possible, thereby not to loose any time with wrong therapy concepts. Therefore a better predictive marker to diagnose steroid-dependant cancer cells is necessary, which is provided by this invention. Prothymosin alpha (PTA) can be measured in tissue samples and plasma of breast cancer and colon cancer patients and indicates, whether the estradiol pathway is functional and consequently whether anti-estradiol therapy may be successful. PTA can be a surrogate for estradiol receptor detection by immune histology, can be a marker to monitor resistance of cancer cells towards anti-estradiol therapy and has the advantage to be determined from easily accessible patient samples such as serum or plasma. Claimed are PTA peptides and nucleic acids, binding agents therefore, test kits and diagnostic and therapeutic methods and compositions.

SEQUENCE LISTING

<110>  BioVisioN AG

<120>  Use of PTA peptides for stratification of individuals having cancer

<130>  PTA

<160>  14

<170>  PatentIn version 3.1

<210>  1
<211>  36
<212>  PRT
<213>  Homo sapiens

<400>  1

Ser Asp Ala Ala Val Asp Thr Ser Ser Glu Ile Thr Thr Lys Asp Leu
1               5                   10                  15

Lys Glu Lys Lys Glu Val Val Glu Glu Ala Glu Asn Gly Arg Asp Ala
            20                  25                  30

Pro Ala Asn Gly
        35

<210>  2
<211>  15
<212>  PRT
<213>  Homo sapiens

<400>  2

Ser Asp Ala Ala Val Asp Thr Ser Ser Glu Ile Thr Thr Glu Asp
1               5                   10                  15

<210>  3
<211>  31

<212>  PRT

<213>  Homo sapiens

<400>  3

Ser Asp Ala Ala Val Asp Thr Ser Ser Glu Ile Thr Thr Lys Asp Leu
1               5                   10                  15

Lys Glu Lys Lys Glu Val Val Glu Glu Ala Glu Asn Gly Arg Asp
            20                  25                  30

<210>  4

<211>  33

<212>  PRT

<213>  Homo sapiens

<400>  4

Ala Ala Val Asp Thr Ser Ser Glu Ile Thr Thr Lys Asp Leu Lys Glu
1               5                   10                  15

Lys Lys Glu Val Val Glu Glu Ala Glu Asn Gly Arg Asp Ala Pro Ala
            20                  25                  30

Asn

<210>  5

<211>  43

<212>  PRT

<213>  Homo sapiens

<400>  5

Ser Asp Ala Ala Val Asp Thr Ser Ser Glu Ile Thr Thr Lys Asp Leu
1               5                   10                  15

Lys Glu Lys Lys Glu Val Val Glu Glu Ala Glu Asn Gly Arg Asp Ala
            20                  25                  30

Pro Ala Asn Gly Asn Ala Glu Asn Glu Glu Asn
        35                  40

<210>  6

<211>  44

<212>  PRT

<213>  Homo sapiens

<400>  6

Ser Asp Ala Ala Val Asp Thr Ser Ser Glu Ile Thr Thr Lys Asp Leu
1               5                   10                  15

Lys Glu Lys Lys Glu Val Val Glu Glu Ala Glu Asn Gly Arg Asp Ala
            20                  25                  30

Pro Ala Asn Gly Asn Ala Glu Asn Glu Glu Asn Gly
        35                  40

<210>  7

<211>  29

<212>  PRT

<213>  Homo sapiens

<400>  7

Ser Asp Ala Ala Val Asp Thr Ser Ser Glu Ile Thr Thr Lys Asp Leu
1               5                   10                  15

Lys Glu Lys Lys Glu Val Val Glu Glu Ala Glu Asn Gly
            20                  25

<210>  8

<211>  8

<212>  PRT

<213>  Homo sapiens

<220>

<221>  MISC_FEATURE

<223>  N-terminal extension  r1 and/or C-terminal extension r2 possible;
        r1 and r2 represent sequences which correspond to the sequence o
       r parts of the sequence of the sequence of PTA from amino acid #
       1 to 8 (for r1) and of amino acid # 17 to 28 (for r2)

<400>  8

Ser Ser Glu Ile Thr Thr Lys Asp
1               5

<210>  9

<211>  8

<212>  PRT

<213>  Homo sapiens



<220>

<221>  MISC_FEATURE

<223>  N-terminal extension  r3 and/or C-terminal extension r4 possible;
        r3 and r4 represent sequences which correspond to the sequence o
       r parts of the sequence of the sequence of PTA from amino acid #
       1 to 22 (for r3) and of amino acid # 31 to 35 (for r4)



<400>  9

Val Val Glu Glu Ala Glu Asn Gly
1               5


<210>  10

<211>  8

<212>  PRT

<213>  Homo sapiens



<220>

<221>  MISC_FEATURE

<223>  N-terminal extension  r5 and/or C-terminal extension r6 possible;
        r5 and r6 represent sequences which correspond to the sequence o
       r parts of the sequence of the sequence of PTA from amino acid #
       1 to 29 (for r5) and of amino acid # 38 to 52 (for r6)



<400>  10

Gly Arg Asp Ala Pro Ala Asn Gly
1               5


<210>  11

<211>  28

<212>  PRT

<213>  Homo sapiens



<400>  11

Ser Asp Ala Ala Val Asp Thr Ser Ser Glu Ile Thr Thr Glu Asp Leu
1               5                   10                  15

Lys Glu Lys Lys Glu Val Val Glu Glu Ala Glu Asn
            20                      25

<210>  12

<211>  35

<212>  PRT

<213>  Homo sapiens

<400>  12

Ser Asp Ala Ala Val Asp Thr Ser Ser Glu Ile Thr Thr Lys Asp Leu
1               5                   10                  15

Lys Glu Lys Lys Glu Val Val Glu Glu Ala Glu Asn Gly Arg Asp Ala
            20                      25                  30

Pro Ala Asn
        35

<210>  13

<211>  110

<212>  PRT

<213>  homo sapiens

<400>  13

Ser Asp Ala Ala Val Asp Thr Ser Ser Glu Ile Thr Thr Lys Asp Leu
1               5                   10                  15

Lys Glu Lys Lys Glu Val Val Glu Glu Ala Glu Asn Gly Arg Asp Ala
            20                      25                  30

Pro Ala Asn Gly Asn Ala Glu Asn Glu Glu Asn Gly Glu Gln Glu Ala
        35                  40                  45

Asp Asn Glu Val Asp Glu Glu Glu Glu Glu Gly Gly Glu Glu Glu Glu
    50                  55                  60

Glu Glu Glu Glu Gly Asp Gly Glu Glu Glu Asp Gly Asp Glu Asp Glu
65                  70                  75                  80

Glu Ala Glu Ser Ala Thr Gly Lys Arg Ala Ala Glu Asp Asp Glu Asp
                85                  90                  95

Asp Asp Val Asp Thr Lys Lys Gln Lys Thr Asp Glu Asp Asp
            100                 105                 110

<210>  14

<211>  111

<212>  PRT

<213>  Homo sapiens


<400>  14

Met Ser Asp Ala Ala Val Asp Thr Ser Ser Glu Ile Thr Thr Lys Asp
1               5                   10                  15

Leu Lys Glu Lys Lys Glu Val Val Glu Glu Ala Glu Asn Gly Arg Asp
            20                  25                  30

Ala Pro Ala Asn Gly Asn Ala Glu Asn Glu Glu Asn Gly Glu Gln Glu
        35                  40                  45

Ala Asp Asn Glu Val Asp Glu Glu Glu Glu Glu Gly Gly Glu Glu Glu
    50                  55                  60

Glu Glu Glu Glu Glu Gly Asp Gly Glu Glu Glu Asp Gly Asp Glu Asp
65                  70                  75                  80

Glu Glu Ala Glu Ser Ala Thr Gly Lys Arg Ala Ala Glu Asp Asp Glu
                85                  90                  95

Asp Asp Asp Val Asp Thr Lys Lys Gln Lys Thr Asp Glu Asp Asp
            100                 105                 110

## Description

The present invention relates to methods for the stratification of individuals suffering from cancer. In particular, the present invention relates to the finding that PTA or fragments thereof allow the discrimination of cancer cells being susceptible to an anti-endocrine therapy from cancer cells not susceptible to said therapy. Thus, PTA can be a surrogate for estradiol receptor detection by immune histology, can be a marker to monitor resistance of cancer cells towards anti-estradiol therapy and to monitor rezidives. PTA has the advantage to be determined from easily accessible patient samples such as serum or plasma. The present invention further encompasses specific PTA peptides and nucleic acids, binding agents therefore, test kits and diagnostic and therapeutic methods and compositions.

### BACKGROUND OF THE INVENTION

The early and correct diagnosis of cancer is of great importance for a successful and effective therapy of cancer. In addition it is crucial to characterize the exact phenotype of cancer cells, which allows to do a stratification of the cancer regarding for example the cancer stage, type of cancer, etc. All of these characteristics of a certain case of cancer are important to choose the best therapy strategy for this patient. For example in the case of breast cancer it is commonly determined, whether the breast cancer cells are positive for the estradiol receptor. This is extremely important for choosing the correct therapy. Estradiol is an important mediator for cell proliferation and consequently estradiol promotes the growth of estradiol receptor positive tumour cells. Therefore estradiol receptor positive cancer cells are usually treated with a so called anti-humoral or anti-endocrine or anti-estradiol therapy. This therapy targets the estradiol signalling pathway for example by blocking the estradiol receptor with analogues of estradiol which bind to the receptor but do not activate it. The most common pharmaceutical used for this purpose is Tamoxifen. Tamoxifen is only used to treat estradiol receptor positive cancer cells, especially estradiol receptor positive breast cancer cells but not for therapy of estradiol receptor negative cancer cells.

The estradiol receptor signalling pathway among others involves a protein termed prothymosin-alpha also known in the literature as PTA, PTalpha, PTα, ProT, ProTalpha, ProTα or PTMA. PTA is a intracellular protein with a sequence length of 110 amino acids and a molecular weight of about 10 to 12 Dalton. PTA among others is involved in chromatin remodelling and it binds to REA (Repressor of Estradiol receptor Activator). Binding of PTA to REA enhances the estradiol receptor-mediated gene transcription (Mol Cell Biol. 2000, 17:6224-32). There are known a few fragments of PTA, namely PTA2-29 which is termed Thymosin 1 (TA1, Tα₁, THNalpha1) and PTA2-36 which is termed Thymosin 11 (TA11, Tα₁₁). The corresponding PTA30-110 and PTA37-110 fragments were only detected in vitro, but not in vivo (J Bio Chem, 2003, 278:13286-93). All amino acid numbers stated in this patent application start with the N-terminal Methionine, which is removed during maturation of PTA. Often in the literature the counting of PTA amino acids is started not with Methionine but with the second amino acid, which is the first amino acid in the processed PTA. TA1 was already identified in 1977 by Goldstein et al. (Proc Natl Acad Sci U.S.A., 1977, 74:725-729). The N-terminal Serine of PTA may be phosphorylated in vivo (Biochemistry, 1993, 32:4587-96). PTA or TA1 have been shown to be altered in their concentrations in humans suffering form various types of cancer including breast cancer (Proc Natl Acad Sci U.S.A., 1993, 90:9504-7, Eur J Cardiothorac Surg, 1997, 12:885-91, Br J Cancer, 1997, 76:1199-209). A clinical study indicated that TA1 may increases the anti-tumour activity of certain immune cells in melanoma and colorectal cancer patients (Int J Immunopharmacol, 1997, 19:493-500).

Legumain is an asparaginyl endopeptidase recently discovered from gene expression profiling and tumour tissue array analysis. Liu et al. analyzed various types of primary human solid tumours and detected Legumain in 75 to 100 % of the analyzed breast carcinoma, colon carcinoma, prostate carcinoma, ovarian carcinoma and central nervous system tumours and in at least 40 % of lung carcinoma, lymphoma and melanoma (Cancer Res., 2003, 63:2957-2864). PTA is processed by Legumain resulting in TA1 or TA11 (J Biol Chem, 2003, 278:13286-93). Legumain has high proteolytic specificy for certain asparaginyl bonds. Preferably Alanine or Proline are present at Position P3 relative to the Asparagine and Glycine at position P1' (P3 = 3 amino acids N-terminal, P1' = one amino acid C-terminal to the Asparagine). The consensus recognition site of Legumain is **Ala/Pro-Xaa-Xaa-Asn-Gly**
and cleavage takes place between Asn and Gly.

### FIELD OF THE INVENTION

The present invention is in the field of stratification, diagnosis and therapy of cancer in individuals, especially of types of cancer with an impaired steroid or estradiol signalling pathway within the cancer cells, especially breast cancer and colon cancer cells. The invention provides prothymosin alpha (PTA) peptides and nucleic acids. Furthermore the invention provides methods to use PTA, PTA peptides and PTA nucleic acids as biomarkers to determine the presence or absence of certain types of cancer cells, methods to determine the type of anti-cancer therapy most likely to be successful and methods to monitor the success of the therapy as well as to determine decreased susceptibility or resistance of the cancer cells towards an anti-endocrine therapy, preferably an anti-estradiol therapy. Furthermore the invention is directed to testkits for the detection of cancer markers or biomarkers and pharmaceutical preparations comprising PTA peptides and nucleic acids.

### SUMMARY OF THE INVENTION

In one aspect the invention provides a method for the stratification of an individual for the treatment of cancer to determine whether the cancer cells of said cancer will be susceptible to an anti-endocrine therapy comprising the steps of:
a) determining the amount of prothymosin-alpha (PTA) or a fragment thereof in a sample of an individual;
b) comparing the result of a) with the result determined using a negative control sample or with an already know reference value, and
c) determining the susceptibility of the cancer cells to an anti-endocrine therapy.

In a further aspect of the invention the amount of PTA or PTA-fragments is altered in cancer cells, which are not susceptible to an anti-endocrine therapy, in comparison to a negative control or in comparison to a reference value, preferably as a consequence of an impaired or disturbed or not fully functional or non functional steroid signalling pathway. Furthermore the PTA-fragment altered in cancer cells not susceptible to an anti-endocrine therapy can be generated by action of the protease Legumain. Preferably the cancer cells suitable for the stratification are cells selected from the group consisting of breast cancer cells, colon cancer cells, central nervous system cancer cells, prostate cancer cells and ovarian cancer cells and preferably the sample used is selected from the group consisting of whole blood, plasma, serum, lymph, nipple aspirate fluid, milk, urine and stool. In a preferred embodiment of the invention the amount of PTA or PTA fragments is altered in cancer cells with an impaired estradiol signalling pathway. Preferably the said PTA or fragments of PTA are determined by a method selected form the group consisting of ELISA, RIA, western blot, protein chip assays, mass spectrometry, immune histology, flow cytometry or by biomolecular methods.
Another aspect of the invention is the use of the methods of the invention for stratification of individuals treated with an anti-endocrine therapy to identify individuals with cancer cells, which acquired a decreased susceptibility or a resistance to said therapy, preferably of individuals treated with a substance selected from the group consisting of anti-estrogenes, estrogene-modulating substances, aromatase inhibitors, Tamoxifen, Toremifen, Raloxifen, Exemestan, Letrozol and Anostrozol. Preferably said stratification is done in combination or in addition to the determination of endocrine receptors, especially estradiol-receptors and/or progesterone receptors.

Another aspect of the invention are peptides, which are fragments of PTA and/or derivatives and/or a polymorphic forms thereof, which peptides are present in altered amounts in a sample of an individual diseased with cancer cells with an impaired steroid signalling pathway as compared to an individual diseased with cancer cells without an impaired steroid signalling pathway with the proviso that said peptides are not TA1 or TA11 (Seq.-ID 11 or 12). Furthermore peptides having a sequence according to Seq.-IDs 1 to 10 as well as polymorphic forms and/or derivatives thereof are another aspect of the invention.

A further aspect of the invention are binding agents specifically binding to a neoepitope of a peptide according to Seq.-ID 1 to 10, which do not bind a peptide according to Seq.-ID 11 to 14 (TA1, TA11, PTA without N-terminal Met, PTA), preferably binding agents which are antibodies or derivatives thereof.

Still another aspect of the invention are nucleic acids encoding a peptide, which peptide is a fragment of PTA and/or a derivative and/or a polymorphic form thereof, which peptide is present in an altered amount in a sample of an individual diseased with cancer cells having a decreased susceptibility or a resistance to an endocrine therapy as compared to an individual diseased with cancer cells being susceptible to an anti-endocrine therapy with the proviso that said peptide is not Seq.-ID 11 to 13 (TA1, TA11, PTA without N-terminal Met) and complementary nucleic acids thereof. Furthermore the invention includes nucleic acid sequence, which hybridizes under stringent conditions to these nucleic acid sequences and which at maximum have a nucleic acid sequence length which is shorter than the nucleic acid sequence coding for Seq.-ID 13 with the proviso that said nucleic acid sequences do not encode TA1 or TA11 (Seq.-ID 11 or 12). Furthermore the invention includes degenerated nucleic acid sequences of said nucleic acid sequence and derivatives and/or fragments of said nucleic acid sequence. In addition the nucleic acid sequences according to the invention can comprise additional nucleic acid sequences, which additional nucleic acid sequences are non-PTA nucleic acid sequences, wherein the non-PTA nucleic acid sequence preferably is a sequence for labelling of the nucleic acid sequence.

Another aspect of the invention are nucleic acids according to the invention with comprise at least one label, which label is not a nucleic acid sequence.

A further aspect of the invention are vectors comprising a nucleic acid sequence of the invention.

Furthermore the invention comprises host cells comprising a nucleic acid sequence or a vector of the invention.

Another aspect of the invention are testkits comprising
a) a peptide which is a fragment of PTA and/or a derivative and/or a polymorphic form thereof, which peptide is present in an altered amount in a sample of an individual diseased with cancer cells having a decreased susceptibility or a resistance to an endocrine therapy as compared to an individual diseased with cancer cells being susceptible to an anti-endocrine therapy with the proviso that said peptide is not Seq.-ID 11 to 13 (TA1, TA11, PTA without N-terminal Met), preferably a peptide having a sequence according to Seq.-ID 1 to 10 and/or a polymorphic form or derivative of said peptide and/or
b) a binding agent specifically binding to a neoepitope of a peptide according to Seq.-ID 1 to 10, which do not bind a peptide according to Seq.-ID 11 to 14 (TA1, TA11, PTA without N-terminal Met, PTA), preferably binding agents which are antibodies or fragments or derivatives of said antibodies, and/or
c) nucleic acid sequences coding for a peptide according to a), complementary nucleic acid sequences thereof, nucleic acid sequences which hybridize under stringent conditions to a nucleic acid according to a) which at maximum have a nucleic acid sequence length which is shorter than the nucleic acid sequence coding for Seq.-ID 13 and with the proviso that said nucleic acid sequence does not encode TA1 or TA11 according to Seq.-ID 11 or 12 and/or
d) nucleic acid sequences which are degenerated with respect to said nucleic acid sequences under c) and derivatives and/or fragments thereof with the proviso that said nucleic acid sequences do not encode TA1 or TA11 according to Seq.-ID 11 or 12 and/or
e) nucleic acids according to c) or d) furthermore comprising at least one non-PTA nucleic acid sequence, wherein said at least one non-PTA nucleic acid sequence preferably is a sequence for labelling of the nucleic acid sequence and/or
f) nucleic acid sequences according to c), d), or e) furthermore comprising a label which label is not a nucleic acid sequence and/or
g) vectors comprising a nucleic acid sequence according to c), d), e) or f) and/or
h) host cells comprising a nucleic acid according to c), d), e) or f) or a vector according to g) and
instructions how to use the test kit for determining the presence, absence, prognosis or relapse of a disorder.

A further aspect of the invention are pharmaceutical preparations comprising any one of the peptides, nucleic acids, vectors or host cells according to a) to h) of the above mentioned testkit or the use of any one of the peptides, nucleic acids, vectors or host cells according to a) to h) of the above mentioned testkit for the manufacture of a testkit or pharmaceutical composition according to the invention.

Another aspect of the invention are testkits comprising
a) any one of the peptides, nucleic acids, vectors or host cells according to a) - h) of the above described testkits and/or
b) a peptide which is PTA or a fragment and/or a derivative and/or a polymorphic form thereof; and/or
c) a peptide according to b) which in addition comprises at least one non-PTA amino acid; and/or
d) a binding agent specific for PTA or specific for a fragment or derivative or polymorphic form thereof; and/or
e) a nucleic acid which is coding for PTA and/or a fragment and/or derivative and/or a polymorphic form thereof; and/or a nucleic acid sequence which is complementary to or which hybridizes under stringent conditions to said nucleic acid; and/or
f) a nucleic acid according to e) which in addition comprises at least one non-PTA nucleotide, and
instructions how to use said testkit for a method or use according to the invention.

A further aspect of the invention are pharmaceutical compositions for treatment and/or prophylaxis and/or diagnosis of a disease selected from the group consisting of breast cancer, colon cancer, central nervous system cancer, prostate cancer and ovarian cancer, comprising any one of the peptides, nucleic acids, vectors or host cells according to a) to f) of the previous chapter. In addition the invention comprises the use of the peptides, nucleic acids, vectors or host cells according to a) to f) of the previous chapter for the manufacture of a testkit and/or a pharmaceutical composition for treatment and/or prophylaxis and/or diagnosis of a disease selected from the group consisting of breast cancer, colon cancer, central nervous system cancer, prostate cancer and ovarian cancer.

### BRIEF DESCRIPTION OF THE DRAWINGS

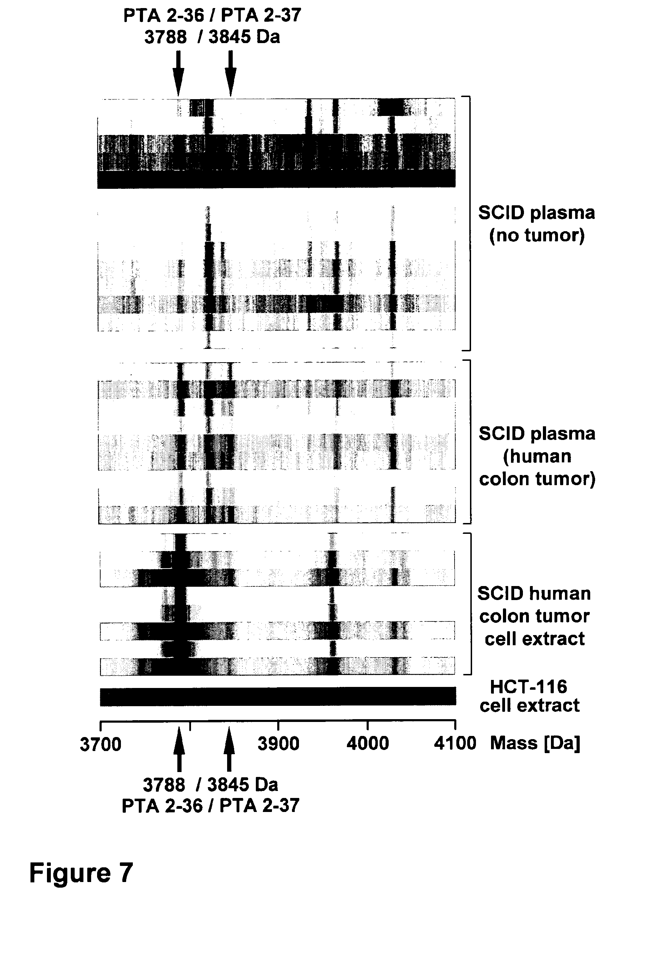

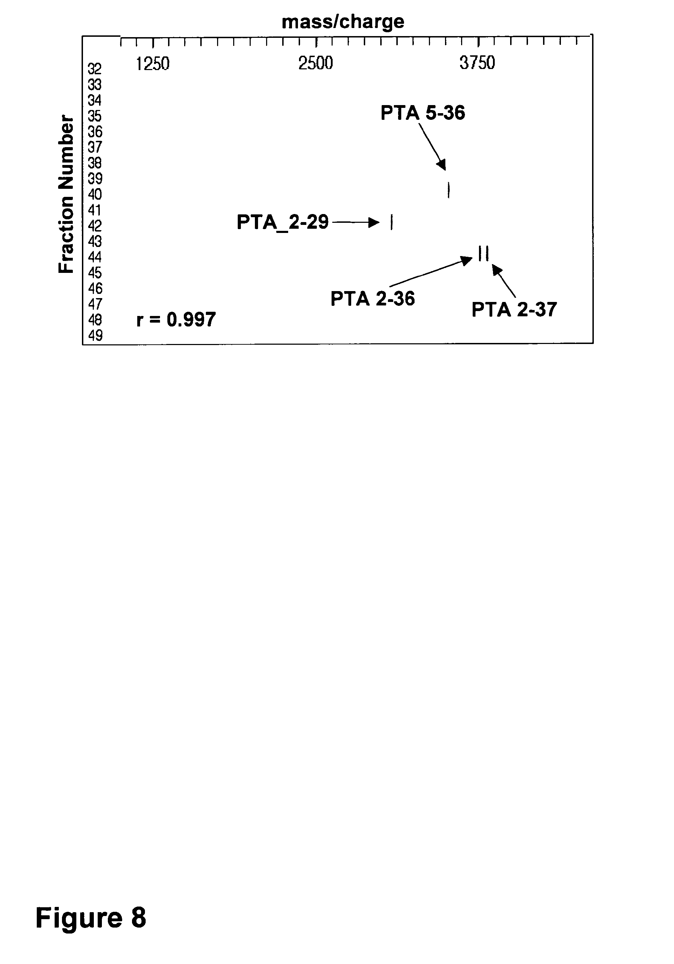

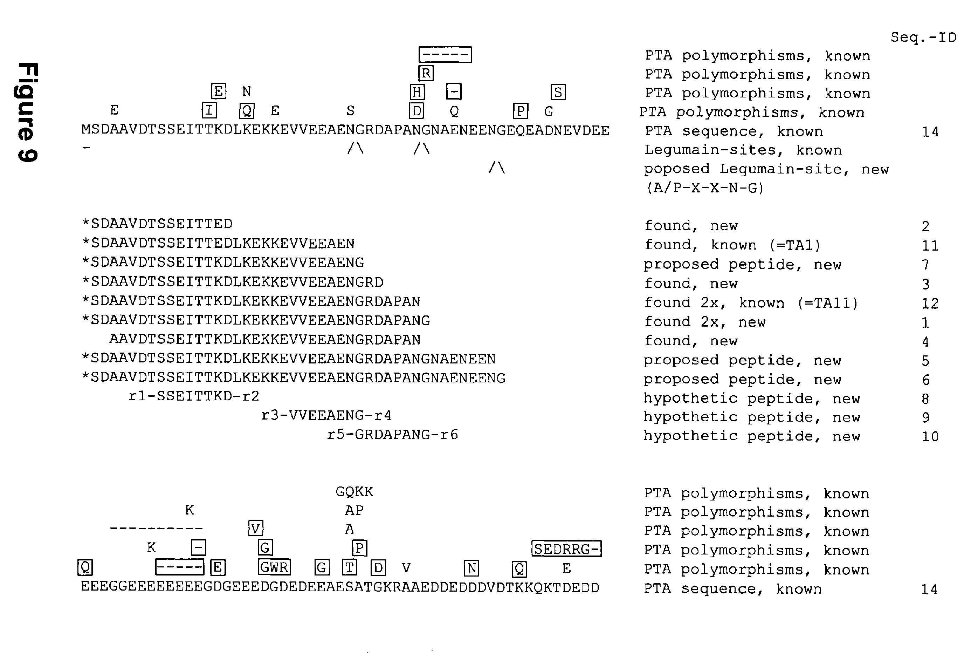

**Figure 1:**
   50 to 800 pmol of neurotensin were introduced into plasma samples, subsequently separated by reversed phase chromatography (RP-chromatography) and those fractions containing the neurotensin were analyzed by mass spectrometry as described in the examples. There is a the linear correlation between the MALDI mass spectrometry signal intensity (y-axis) of the neurotensin peptide and the amount of the neurotensin peptide added to the plasma sample (x-axis), indicating that MALDI mass spectrometry is suitable to quantitatively determine peptides in complex samples such as plasma.
**Figure 2:**
   Box-wisker plots of the MALDI signal intensities of PTA 2-29 (= TA1) and PTA 2-36 (= TA11) measured in estradiol receptor positive (ER+) and negative (ER-) breast cancer tumour samples. The y-axis indicates the MALDI signal intensities of the peptides in arbitrary units.
   The box, i.e. the columns in the diagram, include the range of MALDI signal intensities in which 50% of the respective MALDI signal intensities were found. The lines starting from the box and pointing upward and downward (whiskers) indicate the range in which in each case the 25% of measurements which show the highest signal intensities (upper quarter) are found, and in which the 25% of measurements which show the lowest signal intensities (lower quarter) are found. The full line in the columns indicates the median and the broken line in the columns indicates the mean. Both peptides, PTA 2-29 and PTA 2-36 are present in increased quantities in the estradiol receptor negative breast cancer tumour samples.
**Figure 3:**
   The figure shows the mass spectra of individual fractions obtained by RP-chromatography of primary human breast cancer tumour tissues in a "gel-like" view (MALDI-signal intensity is converted to grey-scale colour). The y-axis represents the MALDI signal intensity and the x-axis represents the molecular mass in Dalton. The arrow at the bottom and at the top of the figure indicate peak representing the peptide of interest (the theoretical, monoisotopic mass was 3103 Dalton; the experimental mass was 3108 Dalton), which, after sequencing, was identified as a fragment of human prothymosin alpha (PTA) corresponding to amino acid residues 2 to 29 of PTA (=TA1, see figure 9, Seq.-ID 11). This signal was present in samples of estradiol receptor negative (ER-) breast cancer tumours, but was absent or very low in estradiol receptor positive (ER+) breast cancer tumours. Four data sets were measured with different numbers of ER+ and ER- tumour samples as indicated in the figure: data set 1 and 2: 28 ER+ and 28 ER-, data set 3: 8 ER+ and 8 ER-, data set 4: 18 ER+ and 20 ER-.
**Figure 4:**

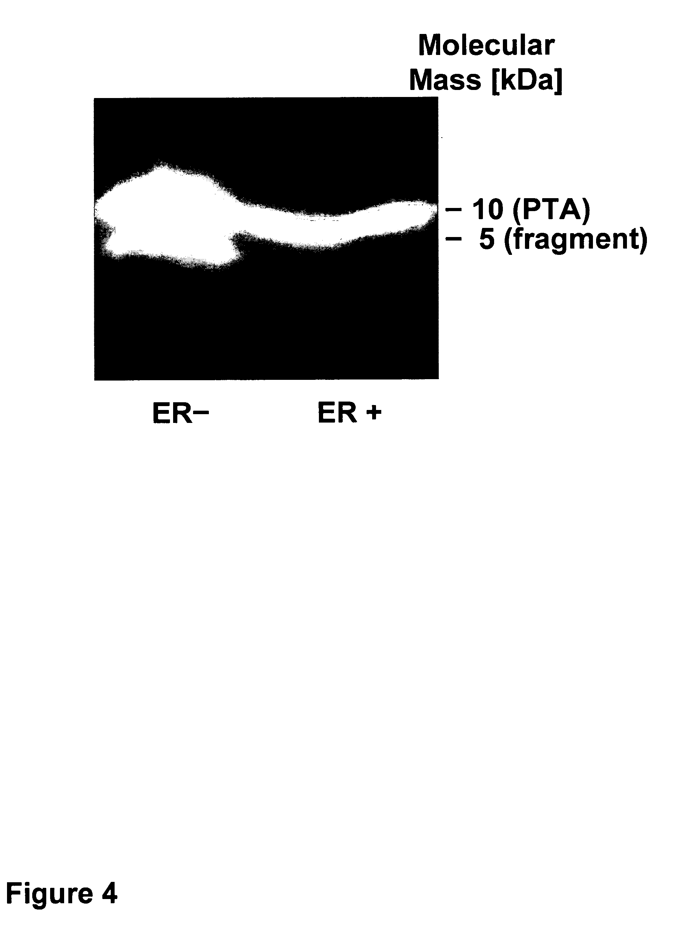

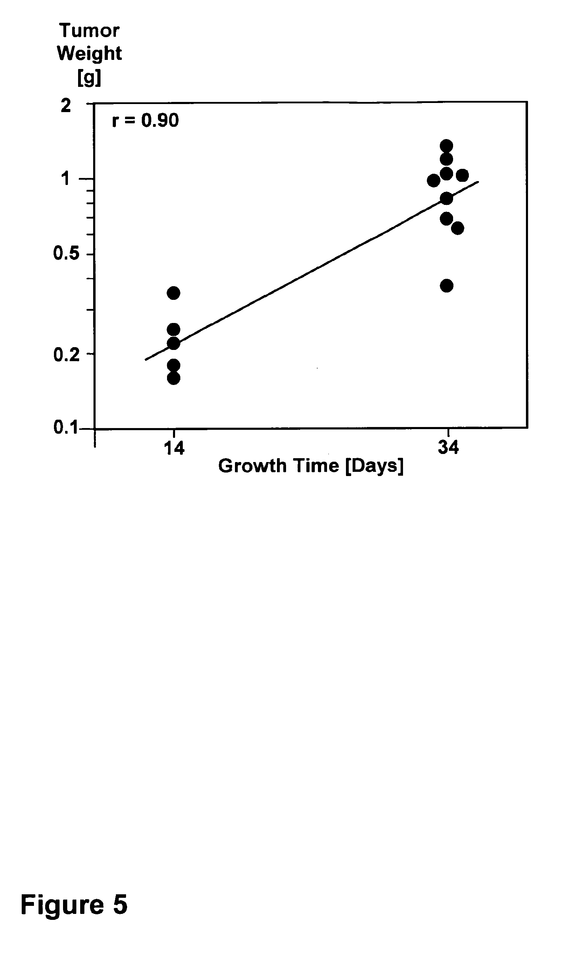

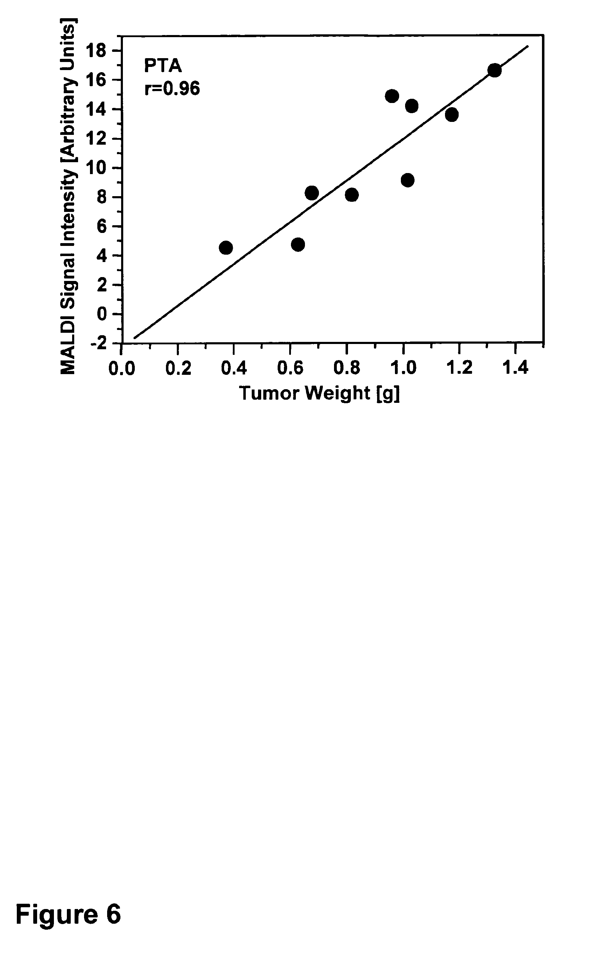

   Figure 4 show a western blot of tissue samples from an estradiol receptor negative breast cancer tumour (ER-) and from an estradiol receptor positive breast cancer tumour (ER+). The antibody recognizes an epitope at the N-terminus of prothymosin alpha (PTA) and consequently recognizes the complete PTA, as well as PTA fragments such as PTA 2-29 or PTA 2-36. Only in the sample of the estradiol negative cancer sample (ER-) there are detected PTA fragments, whereas in both samples the complete PTA (about 10 kDa) is present in about equal quantities.
**Figure 5:**
   The figure shows the tumour weight (y-axis) as a function of growth time of the HCT-116 tumour cells (x-axis) in SCID mice. HCT 116 is a human colon carcinoma cell line which is not proliferating upon estradiol stimulation (Biochem Biophys Res Commun., 2000, 270:425-31). The tumour weight is depicted in gram, the growth time of the tumour is given in days. The measurement values of individual tumours, measured on day 14 or on day 34 post tumour cell injection, are depicted as bullets. The correlation r of time versus tumour weight was 0.90.
**Figure 6:**
   This figure shows the MALDI signal intensity of the peak of the PTA fragment (found in plasma) corresponding to amino acid residues 2 to 37 of PTA (see figure 9, Seq.-ID 1) versus the wet weight of the tumours in individual mice. The x- and y-axis are in logarithmic format. The y-axis represents the MALDI signal intensity in arbitrary units, whereas the x-axis represents the tumour weight. The correlation coefficient r of the MALDI signal intensity versus the tumour volume was r = 0.97.
**Figure 7:**
   The figure shows the mass spectra of individual fractions obtained by RP-chromatography of SCID-mice plasma obtained 34 days after injection of the human HCT-116 colon carcinoma cells in a "gel-like" view (MALDI-signal intensity is converted to grey-scale colour). The y-axis represents the MALDI signal intensity and the x-axis represents the molecular mass in Dalton. The two arrows at the bottom of the figure indicate peaks representing two peptides of interest (theoretical, monoisotopic masses were 3788 and 3845 Dalton; the experimental masses were 3790 and 3846 Dalton), which, after sequencing, were identified as fragments of human prothymosin alpha (PTA) corresponding to amino acid residues 2 to 36 and 2 to 37 of PTA (see figure 9 for sequences; Seq.-ID 12 and 1). These signals were present in the samples of SCID mice with a HCT-116 tumour and in vitro cultured HCT-116 tumour cells, but these signals are absent or very low in plasma samples of SCID mice without a HCT-116 tumour. The MALDI signal intensities of the following samples are shown in the following order: 14 individually measured plasma samples of SCID mice without a HCT-116 tumour, 9 individually measured plasma samples of SCID mice having a HCT-116 tumour, 8 individually measured samples of a HCT-116 cell extract prepared from tumour tissue isolated from SCID mice and 1 sample of a HCT-116 cell extract prepared from in vitro cultured HCT-116 cell.
**Figure 8:**
   Analysis of further peptides which correlate in their MALDI signal intensities with the identified PTA fragments. All measured MALDI mass signals obtained from breast tumour samples were analyzed for correlation with MALDI signal intensities of the identified PTA fragment PTA 2-29. Peptides with a correlation coefficient of r = 0.997 (very high correlation) were chosen for further analysis. The mass and the fraction of the correlating peptides were analyzed by specialized computer software and algorithms (unpublished patent application, file no. EP 04000170.3) to predict, whether these correlating peptides were other fragments of PTA. The following PTA-fragments were identified using this method: PTA 2-36, PTA 2-37 and PTA 5-36. The sequences, masses and fraction number of PTA 2-36 and 2-37 fit to the corresponding data of PTA 2-36 and PTA 2-37 as identified from plasma of SCID mice with a human colon carcinoma tumour (cell line HCT-116) growing in their organism (see figure 7).
**Figure 9:**
   Sequence alignment of the amino acid sequence of human prothymosin alpha (PTA = Seq.-ID 14) with various fragments of PTA. From left to right there is shown: the sequences, a comment regarding the sequence, and the Seq.-ID corresponding to the sequence. Seq.-ID 14 corresponds to the complete amino acid sequence of PTA. In vivo PTA usually is present without it's N-terminal Methionine residue, Seq.-ID 13. On top of the complete PTA sequence there are indicated known polymorphisms of the PTA sequence which are either human polymorphisms (written in boxes) or polymorphisms of the human PTA sequence as compared to the PTA sequences of mice, rats, pigs or cows. A "-" indicates a deleted or missing amino acid residue. If more than one amino acid or "-" or combinations thereof are written without any space in between, these polymorphisms are a single polymorphism affecting more than one amino acid residue at the same time. Combinations of more than one polymorphism are possible. Directly below the complete PTA sequence (Seq.-ID 14) there are indicated 3 cutting sites " Λ " of the protease Legumain, which cuts PTA between amino acid 29/30 and between 36/37 (known from literature) and a new Legumain site at position 44/45, predicted based on the data presented here. Below the complete PTA sequence there are also indicated PTA fragments, which were already known (Seq.-ID 11 and 12), which have been identified (found) in the study presented here (Seq.-ID 1, 2, 3, 4, 11, 12), which are predicted based on the found sequences and other information (Seq.-ID 5 to 7) and which represent hypothetic peptides (Seq.-ID 8 to 10). The " * " indicates that the N-terminal Serine was identified/predicted to be acetylated, "found 2x" indicates, that this peptide was identified in breast cancer and colon cancer samples. The place holders r1 to r6 represent parts of the sequences of PTA. r1 represents 0 to 8 amino acids corresponding to amino acids 1-8 of PTA, r2 represents 0 to 12 amino acids corresponding to amino acids 17-28 of PTA, r3 represents 0 to 22 amino acids corresponding to amino acids 1-22 of PTA, r4 represents 0 to 5 amino acids corresponding to amino acids 31-35 of PTA, r5 represents 1 to 29 amino acids corresponding to amino acids 1-29 of PTA and r6 represents 0 to 15 amino acids corresponding to amino acids 38-52 of PTA. r1 to r6 extend the sequences with Seq.-IDs 8 to 10 with the sequences of PTA corresponding to r1 to r6.

### DETAILED DESCRIPTION OF THE INVENTION

The present invention is directed to the use of certain fragments of prothymosin alpha (PTA) for stratification of individuals having cancer, namely to determine whether in a patient there are present cancer cells having an impaired steroid, especially estradiol or progesterone signalling pathway. In particular, the present invention allows to determine cancer cells being ER positive and being functional in ER signalling, or cancer cells being ER negative or being ER positiv but having a non-functional ER signalling. Although PTA and some distinct fragments of PTA (TA1, TA11) have been described in the context of cancer, PTA or PTA fragments never have been proposed as a cancer marker distinguishing steroid signalling impaired cancer cells from cancer cells with fully functional steroid signalling, especially estradiol- or progesterone-signalling. Also it never has been proposed to use PTA or PTA fragments as an alternative or in addition to steroid receptor, especially estradiol receptor or progesterone receptor detection in cancer tissues by immune histology. As estradiol stimulates the expression of PTA (Endocrinology, 2001, 142:3493-501) it could be expected, that estradiol results in increased PTA fragments in cells with functional estradiol-signalling pathways. Surprisingly we found, that in tissue samples comprising cancer cells with impaired estradiol-signalling and in the plasma of individuals suffering from a tumour comprising cancer cells with an impaired estradiol-signalling PTA fragments are present in increased quantities.

Not to be bound to theory, it is assumed that altered, especially increased, amounts of fragments of PTA are only present in certain cancer cells if a steroid signalling pathway within these cells is impaired, defective or not working the same way as in healthy cells.

Especially in breast cancer and colorectal cancer it is important to know whether the cancer cells have a functional or a not impaired steroid signalling, especially estradiol signalling pathway. In these types of cancer, very often the cancer cells grow, at least in part, as a consequence of steroid- or estradiol-mediated cell proliferation signals. A therapy, which tries to block one of these cell proliferation-promoting pathways can only be successful, if such a pathway is functional in the cancer cells to be treated. Consequently it is very important to make sure that the signalling pathway is present in the tumour cells planned to treat with anti-endocrine, anti-steroid, or anti-estradiol therapy. Commonly the presence of for example a functional estradiol-pathway is proposed, if the immune histologic analysis of a cancer tissue samples reveals the presence of estradiol receptors. This procedure has several disadvantages, which the present invention overcomes. For example immune histologic examinations depend on the subjective impressions, routine, and skills of the examiner which in addition are not directly comparable between different examiners and between different laboratories. In contrast measuring a peptide, such as a fragment of PTA provides a specific value which does not require conversion into a subjective score and therefore is more objective. Furthermore immune histology always depends to some extend on the exact and correct location from which the sample was taken and in addition depends on the very small section of that sample, which is stained for visual inspection. In contrast measuring a peptide from a tissue extract can use the whole tissue sample and therefore gives some kind of an average peptide signal, representative for the whole sample. Especially if not a local tumour tissue, but a more systemic sample such as whole blood, plasma, serum, lymph, etc. are used to determine the peptide, the result is even less dependent from the exact localisation of the tumour. In addition very small tumours or residual tumours after surgery or during or after chemo, radio or other therapies may be difficult to localize and therefore it might be difficult to get a tumour tissue sample at all.

In addition, the claimed methods allow for monitoring the effictiveness of the anti-endocrine therapy, e.g. on a daily basis. Particularly, the methods according to the present invention enable to monitor the effectiveness using readily accessible samples, like blood samples. In addition, the stratification with the methods according to the present invention permits early determination of individuals undergoing anti-endocrine therapy which develop a reduced susceptibility or resistance to the therapy regime.

Another aspect of the methods according to the present invention relates to monitor a relapse of the cancer. Thus, it is possible to determine the recurrence of cancer cells in individuals at an early stage using samples which can be obtained easily.

Furthermore obtaining for example a blood, serum, plasma or urine sample does not require special skills of a medical doctor and can be done by less specialized medical personal such as nurses.

Measurement of a peptide as diagnostic standard, as compared to immune histologic examination is usually easier, cheaper, less susceptible to errors and requires less operator handling, as it can be automated by use of standard techniques such as ELISA, RIA, etc., using robots and computer systems for analysis of the results.

The detection of estradiol receptors in tumour tissue samples by immune histology is the current gold standard to determine, whether anti-estradiol therapy should be used or not. However, a significant number of estradiol-receptor positive tumours are not susceptible to anti-estradiol therapy. One very likely reason for this might be a defective estradiol-pathway, or a defective estradiol receptor, although the estradiol receptor is present. In this case the presence of an estradiol receptor is not sufficient for estradiol-mediated cell proliferation, which therefore cannot be the reason for tumour growth. Consequently an anti-estradiol therapy cannot work. The new method of the invention avoids this problem, as it detects a peptide, originating from a protein, namely PTA, which is necessary in the estradiol pathway and possibly also in other steroid signalling pathways. PTA is a later step in the signal transduction cascade of estradiol as compared to the estradiol receptor. In addition PTA may also be involved in other signalling pathways involved in regulation of cell proliferation, which might be another advantage of determining PTA instead of the presence of estradiol receptors.

Furthermore Legumain, a protease which generates certain fragments of PTA may be itself suitable as a marker for stratification of patients into subgroups of patients, which will benefit from anti-endocrine therapy and patients which will not benefit from anti-endocrine therapy. The same may be true for fragments, derivatives and polymorphic forms of Legumain.

### Amount of PTA or of PTA fragments, reference values and negative control samples

The term "amount" according to the invention means the presence or absence of a substance or the determined quantitative or semi quantitative concentration or quantity of a substance. The "absence" of a substance in general depends on the sensitivity of the method used to determine the amount of the substance. If the amount of a substance is compared to a reference value or to the value determined using a negative control sample, it is defined, that a difference in quantity between both values preferably represents a difference in the value of both data differing more than 1.5-fold, preferably more than 2-fold, preferably more than 3-fold, preferably more than 5-fold, preferably more than 10-fold, preferably more than 20-fold, preferably more than 30-fold, preferably more than 60-fold, preferably more than 100-fold.

A reference value according to the invention, for example a certain reference value for the concentration of PTA2-29 in breast cancer patients can determined empirically. For this purpose the concentration of PTA2-29 can be measured in a series of samples from breast cancer patients for which breast cancer patients the presence or absence of estradiol-receptors in the cancer cells is known. Using these empirical data a cut-off value for the concentration of PTA-29 can be determined, which allows to distinguish estradiol-receptor positive form estradiol-receptor negative breast cancer patient samples. In subsequent measurements of unknown patient samples this cut-off value can then be used to determine whether these unknown patients suffer from an estradiol-receptor positive or an estradiol-receptor negative breast cancer.

If for example it is intended to determine, whether an unknown patient sample originates from an estradiol receptor positive or negative breast cancer sample one can use another patient sample, originating from an estradiol receptor positive breast cancer sample and compare both values measured for PTA2-29. In case estradiol receptor positive breast cancer samples contain less PTA2-29 as compared to estradiol receptor negative samples, and the measured value for PTA2-29 in the unknown sample is higher than the value for PTA2-29 in the estradiol receptor positive sample, which is used as negative control sample, then the unknown sample is a estradiol receptor negative sample. It is also possible to use as a negative control a sample of an individual without breast cancer or of an individual suffering from a type of cancer which in general grows independent of estradiol or a sample from a patient with a non-malignant disease, etc. In general various kinds of negative control samples can be used, as long as it is clear, that these samples do not contain cells, or originated from individuals with cells, which have an altered estradiol pathway.

### Peptides, proteins and PTA

The term "peptide" refers to compounds comprising two or more, preferably 3 or more, 4 or more, 6 or more, 8 or more, 10 or more, 13 or more, 16 more, preferably 21 or more amino acids joined covalently by peptide bonds. Big peptides are commonly termed proteins but in general peptides and proteins are synonyms. The complete amino acid sequence of PTA, as found in nature is meant, if the name of a protein such as PTA is used herein without the addition of the word peptide or fragment, for example PTA and not PTA peptides or PTA and not PTA fragments. PTA fragment is a synonym for PTA peptide. PTA represents the complete amino acid sequence before or after the N-terminal Methionine has been removed. Preferably the peptides and/or proteins and/or fragments of PTA are purified to at least 30 %, preferably 40 %, preferably 50%, preferably 60 %, preferably 70 %, preferably 75 %, preferably 80 %, preferably 85 %, preferably 90 %, preferably 95 %, preferably 97 %, preferably 99 %, preferably more than 99 % purity. The peptide and/or proteins of the invention can comprise mixtures of similar peptides and/or proteins such as derivatives and/or polymorphic forms of the peptide and/or protein, for example a non-glycosilated form of a certain peptide or protein and one or several differently glycosilated forms of said peptide or protein. It is common, that certain modifications such as glycosylations, phosphorylations, etc. are not present in all molecules of an otherwise homogeneous peptide or protein sample, as in nature not all molecules are subjected to these modifications or as some modifications take place only partially or are reversible or as there exists an equilibrium between different derivatives of a certain peptide or protein.

The terms "peptide" and "protein" according to the invention include compounds comprising only amino acids, as well as compounds also comprising non-amino acid constituents such as carbohydrates, lipids, phosphor groups, etc. and include compounds comprising only peptide bonds and compounds also comprising other bonds, e.g. ester, thioester or disulfide bonds. For example it is know in the art, that PTA can be phosphorylated at the most N-terminal Serin residue and that the N-terminal Serine often comprises an additional acetyl-group. Therefore the naturally occurring N-terminus of PTA can be Serine, Phosphoserine, Acetyl-Serine, Acetyl Phosphoryl Serine.

The term non-PTA amino acid or non-PTA amino acid sequence means any amino acid added to the N- or C-terminus of PTA which is not present there in any of the various PTA-sequences occurring in nature, some of which are shown in figure 9.

### Modifications of peptides

Modifications of peptides or proteins according to the invention may comprise modifications due to posttranslational modifications, chemical modifications, enzymatic modifications and modifications due to other mechanisms. Examples of possible modifications include but are not limited to: glycosylation, phosphorylation, sulphatylation, pyroglutamate modification, cystein-disulfide bridges, thioester bridges, methylation, acetylation, acylation, farnesylation, formylation, geranylgeranylation, biotinylation, stearoylation, palmitylation, lipolyation, C-mannosylation, myristoylation, amidation, deamidation, methylation, demethylation, carboxylation, hydroxylation, iodination, oxidation, pegylation, prenylation, ADP-ribosylation, addition of lipids, of phosphatidylinositol, of glycosylphosphatidylinositol (GPI)-anchor, of pyridoxal phosphate, modification of cystein residues resulting in carboxyamidomethylcysteine, resulting in carboxymethylcysteine, or resulting in pyridylethylcysteine, modification of lysine residues resulting in liponic acid, modification of glutamic acid resulting in pyroglutamic acid, etc.

Modifications of peptides or proteins according to the invention may comprise unusual amino acids, chemically or enzymatically modified amino acids etc. including, but not limited to: alpha amino butyric acid, beta amino butyric acid, beta amino iso-butyric acid, beta alanine, gamma butyric acid, alpha amino adipic acid, 4-amino benzoic acid, amino ethyl cysteine, alpha amino penicillanic acid, allysine, 4-carboxy glutamic acid, cystathionine, carboxy glutamic acid, carboxy amido methyl cysteine, carboxy methyl cysteine, cystein acid, citrulline, dehydroalanine, di-amino butyric acid, dehydro amino-2-butyric acid, ethionine, glycine-proline di-peptide, 4-hydroxyproline, hydroxylysine, hydroxyproline, homoserine, homo cysteine, histamine, iso-valeine, lysinoalanine, lanthionine, norvaline, norleucine, ornithine, 2-pipiridine-carboxylic acid, pyroglutamic acid, pyrrolysine, proline-hydroxy proline di-peptide, sarcosine, 4-selenocysteine, syndesine, thioproline, etc. Further examples can be found in databases such as the "Delta Mass" database searchable at the website of the ABRF, the "Association of Biomolecular Resource Facilities": http://www.abrf.org/index.cfm/dm.home?AvgMass=all

### Derivatives, fragments and polymorphic forms of peptides

Derivatives of peptides or proteins are primary characterized by modifications of one or more amino acid residue or modifications of the peptide bond structure, whereas polymorphic forms of peptides or proteins are characterized by differences in the amino acid sequence. It is possible, that a peptide or protein at the same time is a derivative and a polymorphic form of another peptide or protein.

With derivatives of peptides and/or proteins are meant all kind of peptides and/or proteins and/or fragments thereof, including peptides and/or proteins comprising posttranslational modifications, chemical modifications, enzymatic modifications and modifications due to other mechanisms as described elsewhere in this patent application. Derivatives of peptides may comprise amino acid residues different from the standard set of 20 amino acids and/or may comprise peptidomimetic structures.

With polymorphic forms of peptides and/or proteins are meant variations of the sequences due to mutations present in nature (see figure 9) or due to mutations caused by experimental or random manipulation of the sequence using techniques such as molecular biology, genetics or chemistry (nucleic acid or peptide synthesis). Furthermore polymorphic forms of peptides and/or proteins preferably have 70 % sequence identity, preferably 75 %, preferably 80 %, preferably 85 %, preferably 90 %, preferably 95 %, preferably 97 % and preferably have 99 % sequence identity with each others as determined by sequence alignment. An amino acid residue is regarded as identical in two sequences, if within a sequence alignment of these two sequences this amino acid residue is placed at the same position. If for example a first hypothetical sequence "ACDEFGHIKL" and a second hypothetical sequence "ACDEFHIKL" with a deleted "G" are aligned, there would be inserted a gap into the second sequence between "F" and "H" resulting in "ACDEF-HIKL" thereby enabling a better alignment for of the second sequence to the first sequence. As a consequence in the resulting alignment there are now 9 out of 10 positions occupied by identical amino acid residues resulting in a sequence identity of the first to the second sequence of 90 %. Methods how to calculate sequence alignments are given below.

Preferably a peptide or protein which represents a derivative and/or a polymorphic form of another peptide or protein comprises at least 8 amino acid residues, preferably 10, preferably 12, preferably 15, preferably 20, preferably 50, preferably at least 100 amino acid residues, which are present at identical positions within the alignment of both peptides and/or proteins. A peptide, which is a fragment of PTA according to the invention can comprise at least 8 , at least 10, at least 12, at least 15, at least 20, at least 25 amino acids of the PTA amino acid sequence. A PTA fragment, which is a nucleic acid can comprise at least 15, at least 20, at least 30, at least 40, at least 50, at least 60, at least 70, at least 80, at least 90, at least 100 at least 120, at least 150, at least 200, at least 250, at least 300 nucleotides coding for part of the PTA amino acid sequence.

### Anti-endocrine therapy and stratification of cancer or cancer cells

With stratification of individuals or patients, or with stratification of types of cancer or cancer cells according to the invention is meant to divide said individuals or patients or types of cancer or types of cancer cells into sub-groups based on certain characteristics or phenotypes. examples therefore are the stratification of cancer patients into two groups of cancer patients, one which will benefit from an anti-endocrine cancer therapy and another which will not benefit from such a therapy, or the stratification of types of cancer into groups of types of cancer which are sensitive to anti-endocrine treatment and types of cancer not susceptible to such treatment or stratification of cancer cells into cancer cells which have a functional steroid signalling pathway and cancer cells with an impaired steroid signalling pathway. Stratification can be done based on various criteria such as the phenotype of cells, the immunologic properties of cells, the proposed susceptibility of patients to certain medical treatments, etc. Stratification based on phenotypical characteristics or other characterization of types of cancers or cancer cells for example involves to determine, whether they respond to steroids, preferably to estradiol or progesterone. Estradiol and progesterone are steroid hormones which promote growth and proliferation of cells, if these cells possess a functional signaling pathway for these stimuli. Especially in breast cancer, blocking of these stimuli is used in therapy. This kind of therapy is called anti-endocrine therapy. Anti-endocrine therapy in general can use different mechanisms such as blocking of the steroid receptors such as estradiol- or progesterone receptors by binding of ligands to these receptors which do not stimulate the signalling cascade of these receptors but which block these receptors for the natural ligand. These kind of substances are also called SERMs (selective estradiol receptor modulators). Substances in use or in clinical trials for this purpose among others are tamoxifen (brand name: Nolvadex), toremifene (brand name: Fareston), raloxifene (brand name: Evista) and goserelin (Zoladex). Another class of substances for anti-endocrine therapy are estradiol receptor down regulators (ERDs) and one of these substances, Faslodex is currently in clinical trial. A further a approach to anti-estradiol therapy is to lower the amount of estradiol being produced by the body. For example in post-menopausal woman estradiol is no longer produced, but the hormone androgen is converted into estradiol. Certain enzymes termed aromatases, are involved in this conversion of androgen into estradiol and therefore inhibition of aromatases by various mechanisms can be used as anti-endocrine therapy. Currently anastrozole (brand name: Arimidex), letrozole (brand name: Femara) and exemestane (brand name: Aromasin) are in clinical trials for this purpose. Another mechanism, which can be used for anti-endocrine therapy, is the down regulation of steroid receptors such as the estradiol-receptor. An example of such an estradiol receptor down regulator (ERD) is Faslodex, which is currently in clinical trials. In general all of the described and possibly further mechanisms of an anti-endocrine therapy can be used for various kinds of steroids involved in cell proliferation such as estradiol, progesterone and others.

Impaired steroid signaling pathway and determination of an impaired steroid signaling pathway

With impaired steroid signaling pathway according to the invention there is meant, that in cells affected by such an impaired steroid signaling pathway no cellular reaction, especially cell proliferation, takes place upon stimulation with a steroid. It is also possible, that an impaired steroid signaling pathway is only partially defective resulting in decreased but not totally missing cellular reactions such as decreased cell proliferation. Consequently as an example cells with an impaired steroid signalling pathway do not respond to, or do respond to a lesser extend to an anti-endocrine treatment such as a treatment with anti-estradiols such as tamoxifen and are not impaired in proliferation by anti-estradiols or are only partially impaired in proliferation by anti-estradiols. Various methods known in the art can be used to determine, whether a steroid pathway, especially whether an estradiol- or progesterone-signalling pathway is functional in a cell population. The in vitro cultured cells can be stimulated with estradiol or progesterone and the proliferation with and without stimulus can be determined, for example by radioactive 3H-Thymidin incorporation assays or by assays measuring the metabolic activity such as the MTT- (diphenyltetrazoliumbromide) assay, which measures the metabolic activity of the mitochondria or other assays known in the art. Alternatively or in addition the expression of marker proteins or peptides, which indicate a functional steroid signalling can be measured by standard immunologic or molecular biologic techniques such as ELISA, RIA, fluorescence activated cell sorting (FACS), western blotting, polymerase chain reaction (PCR), reverse transcriptase PCR, RT-PCR, Northern blotting, etc. or by alterations in the proteins involved in signalling for example by detection of phosphorylated proteins, or proteins processed or degraded or expression in increased or decrease amounts.

### Preparing of sequence alignments and calculation of sequence homology

To calculate the homology of sequences computer programs such as the GCG software suite (Genetics Computer Group, University of Wisconsin, Madison, WI, USA), including GAP (Nucleic Acids Res. 12:387-95), BLASTP, BLASTN, FASTA (J Mol Biol. 215:403-10) or the well known Smith Watermann-algorithm can be used. Preferred parameter used for amino acid comparisons or alignments comprise the algorithm of Needleman and Wunsch (J Mol Biol. 48:443-53), the BLOSUM 62 matrix (Proc Natl Acad Sci U S A. 89:10915-9), a gap penalty of 12, a gap length penalty of 4 and a threshold of similarity of 0. The GAP software is also suitable to be used with the parameters mentioned. The stated parameters are the default parameter for amino acid comparisons, wherein gaps at the end of a sequence do not decrease the homology value. If very short sequences such as 8 to 20 amino acids long sequences are compared it may be necessary to increase the expectation value up to 100 000 and to reduce the word length down to 2. Further suitable algorithms are the use of gap opening penalties, gap extension penalties and the use of matrixes found in the program handbook, Wisconsin Package, version 9, from September 1997. The choice of the most suitable algorithms depends on the kind of comparisons to perform. If two sequences are compared the GAP or the Best Fit algorithms are preferred, if one sequence is compared to a sequence database the FASTA and BLAST algorithms are preferred. A sequence identity of 70 % is also termed a homology of 70 %. Identity means, that at the same position within both sequences of the alignment the same amino acid residue is present.

### Peptidomimetics

The peptides according to the invention can be prepared or synthesized to represent partially or completely peptidomimetics. This allows to design peptides and/or proteins with more defined properties. For example D-amino acids could be used to prevent proteolysis of peptide bonds. "Peptidomimetics" according to the invention are structures, which mimic the function of amino acids or amino acid sequences. "Peptidomimetics" or "mimetics of peptides" often have additional properties such as increased resistance to proteolysis. Peptidomimetics can replace some or all peptide bonds by other kinds of covalent bonds which can connect amino acids. Peptidomimetics among others can comprise (i) amino acid modifications, (ii) di-peptide analogues, (iii) peptide backbone modifications which for example increase the biological half-life and (iv) secondary structure mimics. Some peptidomimetics for example limit the degree of freedom to the peptide. Examples of amino acid modifications are alpha-C alkylation, alpha-N alkylation, etc., di-peptide analogues peptides with two amino acid side chains connected to each other by covalent bonds, or modifications of the peptide backbone, such as amide bond isosteres or retro-inverso isomers obtained by changing from L- to D-amino acids and inverse N- to C-sequence, etc. Secondary structure mimetics comprise mimetics of structures such as alpha-helices (for example by beta-amino acids), beta-sheets, beta-turns (for example by linchpin structures) and gamma-turns. Further examples of peptidomimetics are spiegelmers® (NOXXON Parma AG, Berlin, Germany) or modifications such as pegylation, which can modify the solubility or immunogenic properties of peptides and proteins.

### Nucleic acids, complementary and degenerated nucleic acids and derivatives

With nucleic acids or nucleic acid sequences according to the invention are meant all kinds of deoxyribonucleic acids (DNA) and ribonucleic acids (RNA) or combinations thereof regardless if these nucleic acid molecules are single or double-stranded, linear, circular or branched. Preferably the nucleic acid molecules are purified nucleic acid molecules which means that the preparation contains at least 50 %, preferably 60 %, 70 %, 80 %, 90 %, 95 %, 99 %, preferably more than 99 % of a certain nucleic acid molecule or of a certain mixture of more than one certain nucleic acid molecule. Examples of nucleic acid molecules are genomic DNA, cDNA (complementary DNA), mRNA (messenger RNA), recombinantly produced nucleic acid molecules, chemically synthesized nucleic acid molecules or enzymatically generated nucleic acid molecules for example by use of polymerase chain reaction (PCR) or nucleic acid molecules purified from natural sources such as prokaryotic or eukaryotic cells, viruses, tissues, organs or whole organisms such as bacteria, yeast, insects, worms, etc. using methods known in the art. Furthermore nucleic acid molecules according to the invention can be partially or completely represent derivatives of nucleic acids which derivatives comprise nucleotides or nucleotide like molecules not found in nature such as phosphorothioates, peptide nucleic acids (PNAs), N3',P5'-phosphoramidates, morpholino phosphoroamidates, 2'-*O*-methoxyethyl nucleic acids, 2'-fluoro-nucleic acids, arabino-nucleic acids, locked nucleic acids (LNA, ribonucleotides containing a methylene bridge that connects the 2'-oxygen of ribose with the 4'-carbon).

Nucleic acids according to the invention furthermore comprise antisense nucleic acids, ribozymes, triplex-forming nucleic acids, RNAi-nucleic acids, nucleic acids suitable as primer for polymerase chain reactions, suitable for in situ hybridization, suitable for hybridization experiments such as Northern or Southern-blots or suitable for nucleic acid chip experiments, nucleic acids suitable as vectors or viral vectors, preferably for expression of a PTA or PTA-fragments or for expression of antisense nucleic acids directed to suppress PTA expression. The nuclei acids preferably have a sequence length of the full length of the mRNA coding for PTA, preferably less than 600, less than 500, less than 400, less than 300, less than 200, less than 100, less than 50, less than 40, less than 30, less than 20, less than 15 nucleotides, depending on the intended use. If the nucleic acid represents an expression vector, the length of the nucleic acid molecule can be much longer up to several thousands of nucleotides as many viruses such as for example bacculoviruses have large genomes resulting in very large vectors. Antisense and triplex-forming nucleic acid molecules preferably have a sequence length of 6 to 50, preferably 10 to 30, 15 to 30, 20 to 30 nucleotides. Ribozymes preferably have sequences hybridizing to the target RNA, which hybridizing sequences have similar length as corresponding antisense nucleic acids and in addition comprise a catalytic ribozyme sequence as known in the art. Nucleic acids for use as primer in polymerase chain reactions preferably have a length of 10 to 60, preferably 15 to 60, 15 to 50, 15 to 40, 15 to 30 nucleotides.

With complementary nucleic acid sequences are meant nucleic acid sequences which hybridize to each other to form a duplex or triplex nucleic acid molecule. The hybridization preferably takes place under stringent experimental conditions which ensure that the hybridization is sequence specific and not random. Stringent hybridizations conditions for nucleic acids are known in the art, and are published for example in Molecular Cloning: A Laboratory Manual, J. Sambrook et al, Cold Spring Harbor, New York, 1998 or in Current Protocols in Molecular Biology, F. M. Asibeö et al, John Wiley & Sons, Inc., New York. An example for stringent conditions for hybridization conditions is the hybridization at 65 °C in a buffer containing 3.5x SSC, 0.02 % Vicoll, 0.02 % Polyvinylpyrrolidon, 0.02 % bovine serum albumin, 2.5 mM NaH₂PO₄ (pH 7), 0.5 % SDS (sodium dodecyl sulphate), 2 mM EDTA (Ethylenediaminetetraacetic acid ). SSC represents a buffer containing 0.15 M sodium chloride and 0,15 M sodium citrate at pH 7. Subsequently to hybridization the membrane with the duplex nucleic acid sticking to it is washed at room temperature with 2x SSC and with 0.1 to 0.5x SSC with 0.1 % SDS at temperatures up to 68 °C. Other protocols for stringent experimental hybridization conditions are known in the art and are within the scope of the invention. A specific hybridization requires that at least 40 %, preferably 50 %, 60 %, 70 %, 80 %, 90 %, 95 %, 99 % of the parts of the nucleic acid sequences hybridizing to each other are complementary to each other. It is not necessary that the complete nucleic acid sequences hybridize to each other, as long as the duplex nucleic acid structure is stable under stringent experimental conditions.

With degenerated nucleic acid sequences is meant that a distinct amino acid sequence can be encoded for by various nucleic acid sequences, as most amino acids are encoded by at least two different codons. For this reason identical amino acid sequence can be encoded by very different sequences of nucleotides.

With non-PTA nucleotide or non-PTA nucleic acid sequence is meant any nucleotide at the 5'- or the 3'-end of a PTA nucleic acid sequence, which is not present at this position in any one of the various PTA nucleic acid sequences present in nature.

### Labelled nucleic acids, peptides, proteins and binding agents

The term "label", as used herein, refers to any moiety that functions to: (i) provide a detectable signal; (ii) interact with a second label to modify the detectable signal provided by the first or second label, e.g. FRET; (iii) affect mobility, e.g. electrophoretic mobility, by charge, hydrophobicity, shape, or other physical parameters, or (iv) provide a capture moiety, e.g., affinity, antibody/antigen, or ionic complexation, resulting in degenerated nucleic acid sequences.

The invention comprises substances such as nucleic acids, peptides, binding agents, antibodies as well as fragments, derivatives and polymorphic forms of said substances, which can comprise a label or other functional group. Suitable are all kinds of labels and functional groups, such as fluorescent molecules, radioactive molecules, luminescent molecules, enzymes, toxins, dyes, metal particles, magnetic particles, polymer particles, biotin or other organic molecules. Especially for mass spectrometry isotope labels or isobaric labels are suitable. Isotope labels can be obtained by use of isotopes with molecular weights different to their natural molecular weight such as oxygen-18, nitrogen-15, deuterium, tritium, carbon-14, sulphur-35, or phosphorus-32 etc. Isobaric labels have the same molecular weight, as long as these labels are intact and have not been fragmented in a mass spectrometer. Therefore isobar labels can more conveniently be analyzed using mass spectrometric methods, as only a limited number of mass differences is present prior to analysis of the sample. Examples of fluorescent molecules are fluorophores such as fluorescein, Texas red, rhodamine, BODIPY, cyber-green, etc, fluorescent proteins or fragments or derivatives thereof such as green fluorescent protein, red fluorescent protein, blue fluorescent protein, renilla fluorescent protein, etc. Examples of radioactive isotopes suitable as label or functional group are phosphorous-32 or -33, sulphur-35, iodine-125, tritium, carbon-14, calcium-45, chromium-51 and all other known radioisotopes which can be coupled to nucleic acids and/or peptides and/or proteins. Examples of enzymes are horse reddish-peroxidase, alkaline phosphatase, luciferase, galactosidase, etc. Suitable toxins are all kinds of toxins such as microbial toxins, toxic peptides or toxic small organic molecules, synthetic toxins or toxins used for medical purposes. An example of a suitable dye is Digoxigenin. Examples of particles are of various particle diameters, preferably between 0.1 to 100 µm. Metal particles are particles can be made from various substances, preferably heavy elements such as gold, silver, platinum or other suitable metals or alloys or mixtures thereof. Examples of magnetic particles are particles preferably made from all kind of magnetic or magnetize able materials including iron, preferably iron oxides such as Fe₃O, Fe₂O₃), Fe₃O₄, etc. Examples of polymer particles are particles made from a polymer such as polyacrylamide, agarose, polypropylene, polystyrene, polytetrafluoroethylene, etc. Various other labels or functional groups, such as biotin, chitin, maltose or glutathione etc. as known in the art, can also be used.

Furthermore labels or functional groups comprising certain additional nucleic acid or amino acid sequences subsequently termed label-sequences can be used. Label-sequences comprising nucleic acid sequences for example can be used for detection of the labelled substance for example by hybridization with a probe specific for the label-sequence or by detection of the label-sequence by polymerase chain reaction, etc. Examples of label-sequences comprising amino acid sequences are his-tags, flag-tags, myc-tags or whole proteins or fragments thereof such as antibodies, glutathione S-transferase, streptavidin, chitin-binding protein, maltose binding protein, various lectins, etc. These labels can be detected by use of antibodies or by use of ligands binding to these labels such as protein A, protein G, protein Y, protein A/G, glutathione, biotin, chitin, maltose or other sugars or these label sequences can function in other ways such as the transport of the labelled substance into the cell, if the label-sequence is for example a HIV-Tat peptide, Antennapedia peptide, VP22 peptide, etc. Label-sequences in general can also be used to purify the label substance by capturing the labelled substance by a binding agent specific for said label-sequence, especially if the label-sequence comprises a amino acid sequence.

### Vectors

Nucleic acid sequences can be part of a vector. A vector according to the invention can be a circular or a linear or branched, single or double stranded, a ribonucleic acid, deoxyribonucleic acid or combinations thereof. The vector can be a naked nucleic acid, a nucleic acid associated with for example proteins, lipids, liposomes, calcium-phosphate precipitates or other substances or combinations thereof. The vector can also be a virus or cell comprising said nucleic acids or vectors. The vector can be suitable to enhance or decrease the amount of for example PTA, PTA peptides, PTA nucleic acids and/or fragments, derivatives or polymorphic forms thereof . Vectors increasing the amount of said PTA, PTA peptides, PTA nucleic acids and/or fragments, derivatives or polymorphic forms thereof are for example transient or stable expression vectors. Vectors decreasing the amount of said PTA, PTA peptides, PTA nucleic acids and/or fragments, derivatives or polymorphic forms thereof for example comprise nucleic acid sequences coding for example for PTA nucleic acid sequence-specific antisense-nucleic acids, ribozymes, triplex-forming nucleic acids, RNAi-molecules, etc. The vector can be present in the form of a virus such as a retrovirus, an adenovirus, a Bacculovirus, a phage or other viruses known in the art. The virus can be a viable virus or a virus needing a helper-virus for proliferation and/or infection. The vector can be unspecific or specific for certain species such as humans, mice, rats, etc. or groups of species such as mammals, rodents, etc., or certain organs or tissues such as muscle-tissue, pancreas, liver, fat-tissue, etc. certain types of cells such as fat cells, muscle cells, cells of the pancreas, neuronal cells, cells from the gastrointestinal tract, etc. or specific for bacteria, yeasts, insect cells, cell lines such as COS-cells or CHO-cells. The specificity of the vector can be due to specific promoter-nucleic acid sequences which are only active in specific cell types or species, or the vector can be specific by mechanisms targeting the vector to specific cells, etc.

### Host cells

Host cell means cells comprising a nucleic acid and/or a vector as described in the previous chapters. The nucleic acid or vector can be present in transient or in stable form in the cell and the nucleic acid or vector can be present in the cytosol or certain organelles of the cell such as the nucleus or mitochondria. The vector sequence or fragments thereof can be present in the cytosol for example in the form of a plasmid or the vector sequence can be partially or completely integrated into the genome of the cell or the genome of the mitochondria, or into a plasmid present in the cell by site (sequence) specific, by random or by other mechanisms.

### Binding agents, antibodies and derivatives

Another embodiment of the invention are binding agents specific for the PTA peptides of the invention. These binding agents may not be specific for human PTA, as the sequences of for example human, murine, rat and bovine PTA are very similar, especially at the N-terminus. Many PTA specific binding agents therefore will also bind to PTA of these and other species. However for diagnostic or therapeutic purposes in for example humans this should not be a problem. Binding agents according to the invention are among others receptors specific for these peptides, phages binding specific to these peptides, spiegelmers® binding specific to these peptides, small organic molecules binding specifically to these peptides, particles or surfaces coated with substances specifically binding to these peptides, such as receptors, phages, small organic molecules, spiegelmers®. Preferably specific binding agents comprise specific antibodies binding said peptides or binding fragments, derivatives or polymorphic forms of said peptides. Also included are antibodies recognizing neo-epitopes of fragments of PTA, which are epitopes present only in fragments of PTA but not in the complete sequence of PTA. Also included are fragments of said antibodies as long as these fragments specifically bind to said peptides. Preferably the binding agent specific for said peptides is an antibody such as an IgG-, IgA-, IgE-, IgD- or an IgY-antibody, which antibody may originate from any species, present in purified form, in form of a cell culture supernatant, an antiserum, as ascites fluid or contained within eggs. The antibodies preferably can originate from humans, mice, rats or other mammals or from eggs preferably from chicken eggs or the antibodies can be produced using recombinant techniques know in the art. The antibody can be a monoclonal, an oligoclonal or a polyclonal antibody. The antibody preferably can be a purified specific antibody, a purified specific antibody further comprising non-specific other antibodies or other proteins such as albumin, an antibody prepared from whole anti-serum, prepared from cell culture supernatant, prepared from eggs or prepared from ascites fluid or an unpurified antibody, preferably unpurified anti-serum, unpurified cell culture supernatant, unpurified ascites fluid or an unpurified recombinant antibody. Preferably a purified antibody or antibody fragment or derivative according to the invention comprises at least 30 %, preferably at least 40 %, at least 50 %, 60 %, 70 %, 75 %, 80 %, 85 %, 90 %, 95 %, or preferably at least comprises 98 % of said antibody and/or antibody fragment and/or derivative thereof.

With specific binding agents are meant for example antibodies, which bind specifically to a peptide ligand such as a PTA fragment, or to a nucleic acid, which binds specifically to a nucleic acid of the invention. The specificity of the binding of an binding agent such as an antibody or an nucleic acid can for example be tested by standard western blotting, northern blotting, reverse transcriptase-polymerase chain reaction (RT-PCR) or other methods known in the art. As samples there can be used for example cells transfected with a nucleic acid coding for a PTA fragment for which the antibody to be tested is supposedly specific. As negative control the same but not transfected cell line or a cell line transfected with a non-PTA fragment can be used. For determining the specificity of antibodies cell lysates from both cell lines can be tested in a western blot. A specific binding agent, such as an antibody, recognizes a band of the correct molecular weight only in the lysate from the PTA-transfected cells but not in the lysate from the negative control. For determining the specificity of nucleic acids, RNA can be purified from both cell lines and tested in for example northern blots or by reverse-transcriptase polymerase chain reaction (RT-PCR). The RT-PCR gives only a PCR product of correct size, using the RNA isolated from the PTA-transfected cell line. In a Northern blot, a specific binding nucleic acid recognizes a band of the correct molecular weight only in the RNA sample of the transfected cell line but not in the RNA from the negative control. However, as known in the art, there can also be signals in the negative control, but the signal in the negative control is clearly lower, if the binding agent is specific for the ligand transfected into the cell line. The specificity can be further tested by increasing the stringency of the washing steps in the northern blots, which should gradually reduce the signal in the negative control, whereas the signal of the PTA-transfected cells remains present.

The invention furthermore comprises derivatives of antibodies such as an antibody comprising parts of antibodies originating from different species, for example humanized antibodies, which for example comprise the antigen binding domain of a murine antibody and other parts of the antibody or antibody fragment originate from human antibodies. The invention also includes fragments of antibodies as long as these antibodies still specifically bind to PTA fragments or derivatives thereof. Examples of possible antibody fragments are among others Fab2- or Fab antibody fragments. Further derivatives of antibodies according to the invention are antibodies or fragments of antibodies covalently or non-covalently bound to other substances such as organic compounds, inorganic compounds, peptides or proteins. Examples for such derivatives of antibodies are antibodies labelled with radionuclides, toxins, enzymes, fluorophores, fluorescent proteins, dyes, or label sequences such as the label sequences described in previous chapters.

### Pharmaceutical compositions

Another embodiment of the invention are pharmaceutical compositions. These pharmaceutical compositions comprise substances such as PTA peptides or fragments, derivatives or polymorphic forms of said peptides, or nucleic acids coding for said peptides or fragments, derivatives or polymorphic forms of said nucleic acids or complementary or degenerated sequences, vectors comprising said nucleic acids, host cells comprising said nucleic acids or said vectors.

The pharmaceutical compositions of the invention can be used for therapy, prophylaxis, treatment of a relapse of cancer, specially of cancer cells with an altered steroid signalling pathway, especially an altered estradiol signalling pathway, especially breast cancer or colon cancer.

The pharmaceutical compositions can be administrated orally, sublingually, injected intra venous, injected subcutaneously, injected into a tumour, into muscle, fat or other tissues, administered topical onto the skin or onto a mucosa, inhaled as aerosol into the lungs or administrated rectal or through other routes. The pharmaceutical composition can be supplied as a tablets, pills, capsules, powders, granulates, salves, plasters releasing substances transdermale to the individual, tinctures, uvula, suspensions, solutions, preferably in sterile physiological sodium chloride solutions for use in injections or as continuous infusions, etc. The pharmaceutical compositions can comprise additives such as filling additives, antimicrobial additives, preservative additives, colouring additives, flavouring additives, smelling additives, protective or stabilizing additives, etc. Preferred protective or stabilizing additives for example are substances which inhibit degradation of peptides and proteins for example protease inhibitors or carrier proteins such as albumins, preferably human albumins, to protect the peptides and proteins from degradation, aggregation or from loss of activity. The pharmaceutical compositions can be applied to an individual in need once or repeatedly. It can be applied one or more times a day, weekly, monthly or in longer time intervals. The pharmaceutical compositions can be used alone or in combination with other pharmaceutical compositions. Combined pharmaceutical compositions can have less than additive, can have additive and can have synergistic effects on the individual treated or tested. The pharmaceutical composition can comprise up to 3 µg, up to 10 µg, up to 30 µg, up to 100 µg, up to 300 µg, up to 1 mg, up to 30 mg, up to 300 mg or more than 300 mg of PTA-peptide, nucleic acid coding for said peptide, binding agent specifically binding to said peptide, antibody specifically binding to said peptide or of a fragment or derivate or polymorphic form of the said or combinations thereof.

If the pharmaceutical composition is used as a diagnostic composition it can be used alone or in combination with other diagnostic compositions or diagnostic methods. The result of the diagnosis by combination of two or more pharmaceutical compositions and/or diagnostic methods can improve the reliability of the diagnosis less than additively or they can improve it additively or they can improve the diagnosis synergistically. The pharmaceutical composition can be used for diagnosis to prove the presence or to prove the absence of a cancer cells, preferably of cancer cells with an impaired steroid, preferably with an impaired estradiol signalling pathway, preferably of breast cancer or colon cancer cells.

### Suitable methods to analyse the sample

Generally all methods suitable to detect and analyse the peptides present in the sample can be used in the methods of the invention and can be used to determine presence, absence or quantity of the peptides, nucleic acids and fragment, derivatives of the invention. Preferably mass spectrometric, protein chip assays, immunology and molecular biology methods can be used.

Suitable mass spectrometric methods among others are matrix assisted laser desorption ionisation (MALDI), continuous or pulsed electrospray ionization (ESI) and related methods such as ionspray or thermospray or massive cluster impact (MCI). The ion sources can be matched with detection formats including linear or non-linear reflection time-of-flight (TOF), single or multiple quadrupole, single or multiple magnetic sector, fourier transform ion cyclotron resonance (FTICR), ion trap, and combinations thereof, e.g. ion-trap/time-of-flight. For ionisation, numerous matrix/wavelength combinations (MALDI) or solvent combinations (ESI) can be used. Other mass spectrometric methods suitable are for example fast atom bombardment (FAB) mass spectrometry, Surface Enhanced Laser Desorption/Ionisation (SELDI) mass spectrometry, isotope coded affinity tag (ICAT) mass spectrometry, or affinity mass spectrometric methods.

Furthermore suitable immunologic methods among others are enzyme linked immuno assays (ELISA), sandwich, direct, indirect, or competitive ELISA assays, enzyme-linked immunospot assays (ELISPOT), radio immuno assays (RIA), flow cytometry assays (FACS = fluorescence activated cell sorting), immunohistochemistry, western blot, fluorescence resonance energy transfer (FRET) assays, protein-chip assays using for example antibodies, antibody fragments, receptors, ligands, or other binding agents specific for the peptides of the invention.

Among others Northern or Southern blot hybridization, nucleic acid dot- or slot-blot hybridization, in situ hybridization, nucleic acid chip assays (for example using RNA, DNA or other nucleic acids to specifically capture nucleic acid of interest), polymerase chain reaction (PCR), reverse transcriptase PCR (RT-PCR), real time PCR (taq-man PCR) can be used as molecular biology methods to detect the nucleic acids of the invention.

Further methods such as nuclear magnetic resonance (NMR), fluorometry, colorimetry, radiometry, luminometry, or other spectrometric methods, liquid chromatography, capillary chromatography thin-layer chromatography, plasmon resonance (BIACORE), one- or two-dimensional gel electrophoresis, etc. can be used to detect the peptides or nucleic acids of the invention.

### Test kits

A further aspect of the invention are test kits for determining the absolute concentration or the relative concentration or for determining the presence or absence of a peptide or nucleic acid of the invention, or a fragment or derivative or polymorphic form thereof. The test kit can be used to diagnose the absence or presence of cells with a functional steroid signalling pathway, preferably a functional estradiol-signalling pathway. The test kit further can be used to predict the occurrence or to predict the grade of stage of the cancer and/or to predict and/or monitor the success of a therapy for said cancer and/or predict and/or monitor a relapse of a said cancer. Preferably the test kit can be used to early diagnose and/or predict said cancer, preferably before the diagnosis is possible by other diagnostic tests which are known in the art. The test kit can be used to analyze any kind of sample from an individual including but not limited to whole blood, blood cells (such as leukocytes, B-cells, T-cells, monocytes, macrophages, erythrocytes and thrombocytes), serum, plasma, hemofiltrate, nipple aspirate liquid, urine, lymph, lymph knot tissue, stool, liquor cerebrospinales, tissue from the colon or the rectum or other tissues and organs as well as combinations thereof. The test can be done with pooled samples of one or more than one individual. The sample can be a pre-processed sample, for example a sample fractionated by chromatography, filtration, precipitation, liquid extraction or other extraction method, immunoprecipitation, etc. Subsequently individual or two or more combined fractions, originating from one or more than one sample can be analyzed. The test can be done once or several times, preferably several times over a period of time to analyze the time course of quantity changes of PTA, of a PTA peptide, a PTA nucleic acid or a fragment, derivative or polymorphic form thereof. The test can be done with various types of test kit known in the art including but not limited to the methods of analyzing a sample as described elsewhere in this patent application. The test kit can comprise substances such as PTA, PTA-peptides, PTA nucleic acids, PTA binding agents, PTA-specific antibodies or fragments, derivatives or polymorphic forms of the said or combinations thereof. Said substances can be present in the kit in labelled form or in a form enabling their labelling, or in a form facilitating their detection and/or quantification.

The PTA, PTA-peptides, PTA nucleic acids and/or fragments, derivatives or polymorphic forms of the said can be used as standard or as capture agent able to bind to antibodies present in the sample to be tested or can be used as labelled ligand of a binding agent competing for binding to a binding agent with the same or similar ligands present in the sample to be tested.

The kit can comprise agents binding or hybridizing specifically to said PTA, PTA peptides or PTA nucleic acids. Furthermore the test kit of the invention can comprise instructions how to use the test kit, how to prepare the samples, what kind of samples to use, how to analyze and interpret the results, etc. Especially the test kit can comprise instructions, how to use the kit for determining the presence or absence of cancer cells with an impaired steroid signalling, preferably estradiol or progesterone signalling pathway, preferably breast cancer or colon cancer cells with such an impaired signalling pathway. The instructions for the test kit can be in written form, presented as pictures, films, audio, computer-multi media or any other format suitable to explain, how to use the kit.

### New uses for identified markers

Some of the identified peptides are PTA fragments which are known in the prior art (TA1, TA11), but peptides which are PTA fragments or even the complete PTA with or without N-terminal Methionine residue had never been described before as marker suitable for stratification of indivuduals with cancer cells, namely to determine whether said cancer cells are sensitive towards an anti-endocrine or anti-estradiol therapy.

### EXAMPLES

The following examples are intended to illustrate the invention, however they are not meant to limit the scope of the invention in any way.

### Example 1: Collection of primary human breast cancer tissue samples and sample preparation

Primary human breast cancer tissue was collected during biopsy or surgical removal of tumour tissue. Use of the primary human tissue for this study was approved by the local ethics committee of the Medizinische Hochschule Hannover, Germany and all patients gave written approval for use of their tissue samples in this study. 20 µm slices of tumour tissues, which were left over after immune histologic determination of the estradiol receptor presence or absence were stored at -80 °C until further use. After quantification of the tissue samples by weighing, the tissue samples were incubated in extraction buffer (6 M Guanidin-Hydrochlorid, 50 mM Glycin, pH adjusted to 2.6 by use of HCl) for 10 min. at 99 °C and 1100 rpm in thermo mixer. The "concentration" of tissue in extraction buffer was 20 mg/mL. After centrifugation for 15 min. at 18 000x g at room temperature the supernatant was harvested and subjected to ultrafiltration or stored at -80 °C until further processed. Ultrafiltration cartages (Microcon® YM-50, cut-off 50 000, order # 42416, Millipore GmbH, Schwalbach, Germany) were rinsed with 400 µL water by centrifugation for 1 min. at room temperature at 12 500x g and up to 400 µL tissue extract were loaded per cartage. After centrifugation for 1 h at 12 500x g at room temperature the filtrate was collected and further processed or stored at -80 C° for later use. The retenate was not used for further analysis.

### Example 2: Westernblotting using primary human breast cancer tissue samples

Primary human breast cancer tissue samples, stored at -80°C were mixed with lysis buffer (50 mM HEPES, 0.1 % Tween-20, 20 mM KCl, 1 mM dithiothreitol) and homogenized using a ultrasonic device. After centrifugation the pellet was discarded and the supernatant collected in fresh tubes. The protein concentration of the tissue cell lysate was determined using a common protein determination assay (Bradford assay) as known in the art. The tissue cell lysate was diluted with concentrated sample buffer resulting in a final concentration of 15 µg protein in a total volume of 10 µl. After heating the samples for 5 min. at 95 °C the samples were separated using pre-casted SDS-polyacrylamide gradient gels (Bio-Rad Laboratories GmbH, Munich, Germany, order # 161-1178). Proteins and peptides present in the sample were separated for 45 min. at 25 mA electric current and subsequently transferred to Nytran SuPerCharge membrane (Schleicher und Schuell GmbH, Dassel, Germany) using standard semi-dry blot procedures as known in the art. Using a transfer buffer containing Tris/Glycine/Methanol according to the manufacturer's instructions the transfer took place for 1 h at 75 mA current. The protein molecular weight marker used was PageRuler (Fermentas GmbH, St. Leon-Rot, Germany, order-no. #SMO671). After protein transfer the membrane was blocked over night using phosphate buffered saline (PBS) containing 5 % (weight per volume) skim milk powder. The membrane was washed 3 times for 10 min. each using PBS, incubated for 1.5 h at room temperature using an anti-PTA antibody (anti-Prothymosin alpha (NT) (2F11) antibody, order # ALX804-486-C100, Alexis Deutschland GmbH, Grünberg, Germany) diluted 1:200 in PBS, washed 3 times for 10 min. each with PBS, incubated for 1.5 h at room temperature with a 1:5000 diluted secondary antibody (horseradish peroxidase-labelled goat-anti-mouse antibody) and finally washed 3 times for 10 min. with PBS. Detection of bound antibodies was done by incubating the membrane with detection solution (Tris-HCl, Luminol, Cumarin, H₂O₂) for 10 min. and subsequent photographing using a CCD camera. The used antibody, according to the manufacturer, recognizes an epitope which includes the amino acids 1 to 31 of human PTA.

### Example 3: Growth of tumour cells, injection into mice and sample collection

The human colorectal carcinoma cell line HCT-116 was obtained from ATCC, Manassas, VA, USA, order no. CCL-247. The tumour cells were cultured in RPMI 1640 medium supplemented with 10 % fetal bovine serum and 2 mM L-glutamine at 37 °C with 5 % CO₂. All tissue culture media and supplements were from Gibco BRL, Gaithersburg, MD , USA. Cell passaging of the adherent tumour cells was performed by splitting the cell culture twice a week at a ratio 1:10. At the day of injection (time point zero) cells were harvested from the culture flasks using Trypsin / ethylenediaminetetraacetic acid (EDTA) solution as known in the art and transferred into culture medium, washed once and resuspended in phosphate buffered saline (PBS). After an additional washing step with PBS the final cell concentration was adjusted to 15 000 000 live cells per mL. The tumour cell suspension was carefully mixed to avoid cell aggregation, kept on ice and drawn into a 1.0 mL syringe without attached needle. 200 µL cell suspension (containing 3 000 000 cells) were subcutaneously injected into the right lateral flank of the mice using a needle with a size of 0.45 x 25 mm. A total of 60 mice were injected with tumour cells. 40 additional animals served as controls and were injected with PBS only. The animals with a tumour were sacrificed on days 7, 13 or 34 after tumour cell injection. Control groups of 20 animals each were sacrificed on days 7 and 34. EDTA-blood was obtained by cardiac puncture at the time points indicated. EDTA plasma was prepared as known in the art and samples from individual animals were stored at -80 °C within 30 minutes after blood drawing. Weights and volume of the explanted primary HCT-116-tumours were determined at the time point of dissection. The tumour volume was calculated by multiplying the length and the squared average width measured in centimetres resulting in millilitre (mL) of tumour volume. Immediately after weighing and determining the volume the individual tumours were frozen in liquid nitrogen and stored at -80 °C until further processed.

### Example 4: Sample preparation using murine plasma

Prior to analysis a reduction of the protein load of the plasma samples was achieved by trichloroacetic acid (TCA) precipitation as known in the art. Samples of 0.5 mL plasma were added to 1 mL chilled, distilled water. For protein precipitation 0.5 mL TCA (20 % (v/v) concentration of TCA) were added and the tubes vigorously mixed for 30 seconds. After incubating for 30 minutes on ice, samples were centrifuged at 18 000 g for 30 minutes at 4 °C. The supernatants were transferred into new tubes and stored at -80 °C until further processed. A 375 µL-equivalent of plasma was used per chromatographic run.

### Example 5: Sample preparation using HCT-116 tumours, isolated from SCID mice

The mass of the samples was determined by weighing. After addition of 80 µL of 200 mM acetic acid to 4 mg moist mass of tumour tissue, samples were boiled for 10 minutes. Typically 20 to 50 mg tumour tissue were used as staring material. Subsequently the pH of the sample was adjusted to pH 2 to pH 3 using a 30 % stock solution of HCl and finally the sample was diluted to 1.5 mL using a 0.1 % stock solution of TFA. After 10 minutes centrifugation at 18 000x g at 4 °C the supernatant was harvested and stored at -80 °C until further processed. Cell extracts, corresponding to 4 mg tumour tissue were used per chromatographic run.

### Example 6: Sample preparation using in vitro cultured cells

Cells were removed from tissue culture plates by use of trypsin/EDTA and a rubber policeman, centrifuged for 5 minutes at 600 g at room temperature and washed twice with PBS. For peptide extraction moist mass standardized samples of 50 000 to 400 000 cells were used. After addition 200 µL of 200 mM acetic acid the samples were boiled for 10 minutes. Subsequently the pH of the samples was adjusted to pH 2 to pH 3 using a 30 % stock solution of HCl and finally the samples were diluted to a final volume of 1.5 mL using a 0.1 % stock solution of TFA. After 10 minutes centrifugation at 18 000 g at 4 °C the supernatant was harvested and stored in tubes at -80 °C until further processed. Cell extracts, corresponding to 50 000 to 400 000 cells were used per chromatographic run.

### Example 7: Collection and preparation of human plasma

Plasma samples from 19 male colon carcinoma patients of various cancer stages, having a mean age of 62 years were collected by standard methods known in the art using EDTA as anti-coagulant. The collection tubes were centrifuged for 10 min. at 2000 g and 4 °C. Plasma samples were stored at -80 °C until further processed. Prior to chromatography 4 mL of a pool of these 19 plasma samples were subjected to trichloroacetic acid (TCA) precipitation as known in the art. In detail aliquots of 500 µL plasma were added to 1 mL chilled, distilled water. Protein precipitation was carried out by adding 500 µL of ice cold 20 % (v/v) TCA and vigorously mixing of the tubes for 30 seconds. After incubating for 30 min. on ice, the tubes were centrifuged for 30 minutes at 18 000 g and 4 °C. The supernatants were used for further analysis. TCA precipitation was done to remove high molecular weight proteins and to enrich peptides and low molecular weight proteins. Subsequently the supernatant of the TCA precipitation was separated into 96 fractions as described in example 6. A second fractionation using a 3.6 mL plasma equivalent of fraction 42 of the first fractionation (according to example 6) was done using a Jupiter C18 reverse phase column with a size of 2 x 250 mm (Phenomenex, Aschaffenburg, Germany). The chromatography took place at 33 °C using a HP 1100 HPLC with a micro flow cell both supplied by Agilent Technologies, Böblingen , Germany. Buffer A was 0.06 % (v/v) trifluoroacetic acid (TFA) and 99.94 % (v/v) distilled water and buffer B was 0.05 % (v/v) TFA, 80 % (v/v) acetonitrile and 19.95 % (v/v) distilled water. The chromatographic conditions were as follows:
- 5 % buffer B during the first minute
- a linear gradient from 5 to 15 % of buffer B during the next 2 minutes
- a linear gradient from 15 to 40 % of buffer B during the next 42 minutes
- a linear gradient from 40 to 100 % buffer B for the next 4 minutes
- 100 % buffer B during the next 4 minutes
- a linear gradient from 100 to 50 % buffer B during the next 1.5 minutes
- a linear gradient from 50 to 100 % buffer B during the next 3 minutes
- and finally a linear gradient from 100 to 50 % buffer B during the final 2.5 minutes.

The flow rate was 100 µL/min. and 96 fractions, each containing of µL were collected during the gradients from 5 to 100 % buffer B.

### Example 8: Liquid chromatography of the samples

The separation method carried out was reverse phase chromatography. Various RP chromatography resins and buffers are equally suitable. The separation of peptides and/or proteins using a C18 reverse phase chromatography column having a size of about 4 mm x 250 mm was done as described below. Preferably a Source RPC, 4,6 x 150 mm (Amersham Biosciences Europe GmbH, Freiburg, Germany) is used. Buffer A was 0.06 % volume per volume (v/v) trifluoroacetic acid (TFA) and 99,94 % (v/v) distilled water and buffer B was 0.05 % (v/v) TFA, 80 % (v/v) acetonitrile and 19.95 % (v/v) distilled water. The chromatography took place at 33 °C using a HP 1100 HPLC with a micro flow cell both supplied by Agilent Technologies, Böblingen , Germany. The pH of the sample was adjusted to pH 2 to pH 3 using a stock solution of 30 % (v/v) HCl and subsequently the samples were diluted to 1.5 mL using a 0.05 % (v/v) stock solution of trifluoroacetic acid. Prior to chromatography the samples were centrifuged at 4 °C and 18 000 g for 10 minutes and finally 1.5 mL of sample were loaded onto the chromatography column representing an equivalent of 375 µL plasma, 50 000 to 400 000 in vitro cultured cells, 4 mg moist weight of tumour tissue or 1.5 mL cell culture supernatant. Buffers A and B were mixed in various ratios during chromatography and subsequently only the percentage of buffer B is stated. The percentage of buffer A is 100 % minus % buffer B (v/v). The chromatography conditions were as follows:
- 5 % buffer B for 1 min,
- a linear gradient from 5 to 50 % buffer B during the next 44 min.
- a linear gradient from 50 to 100 % buffer B during the next 4 min.
- 100 % buffer B for the next 4 min.

The flow rate was 500 µL/min. and 96 fractions, each containing 0.5 mL were collected during the gradients from 5 to 100 % buffer B.

### Example 9: Mass spectrometry of the samples

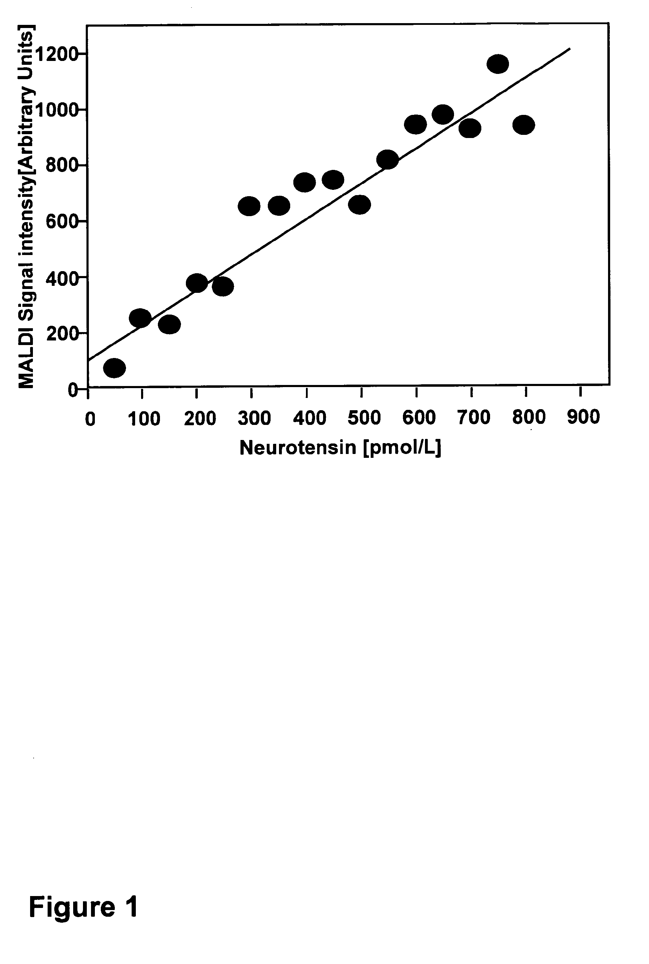

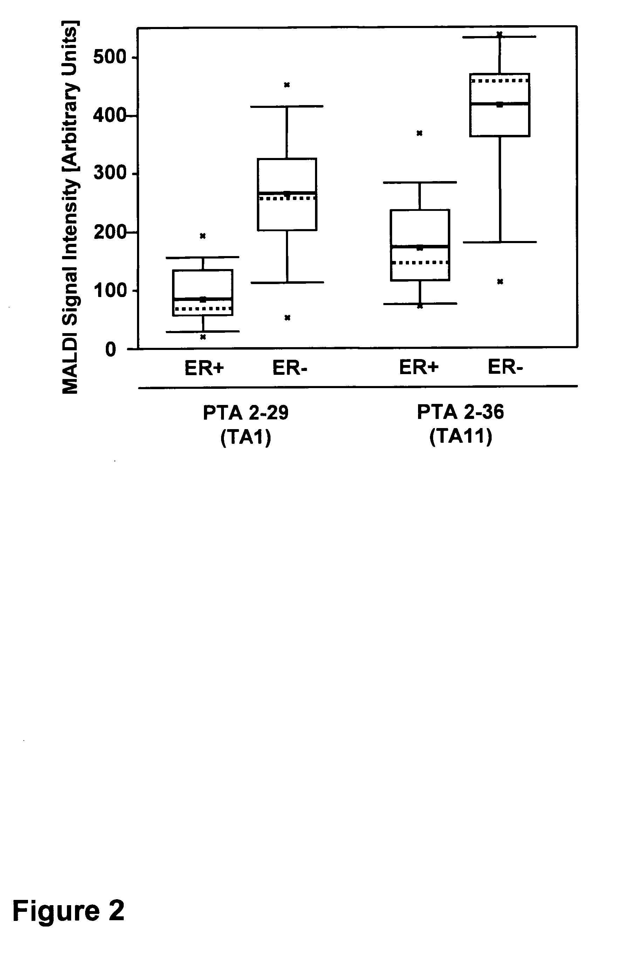

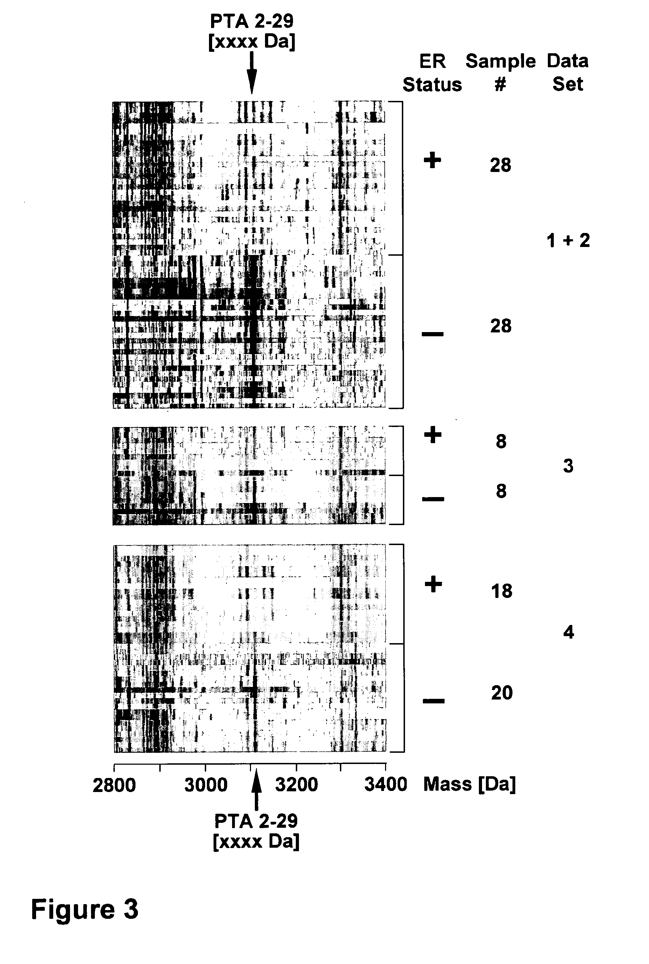

For mass spectrometric analysis, typical positive ion spectra of peptides were produced in a MALDI-TOF (matrix-assisted laser desorption ionization time of flight) mass spectrometer. Suitable MALDI-TOF mass spectrometers among others are manufactured by PerSeptive Biosystems, Framingham, USA (Voyager-DE, Voyager-DE PRO or Voyager-DE STR) or by Bruker Daltonik, Bremen, Germany (BIFLEX). Typical matrix substances suitable for peptides are matrix substances which comprise an organic acid such as 3,5-dimethoxy-4-hydroxycinnamic acid, alpha-cyano-4-hydroxycinnamic acid and 2,5-dihydroxybenzoic acid. For MALDI sample preparation we used alpha-cyano-4-hydroxycinnamic acid as matrix and 6-desoxy-I-galactose as co-matrix, dissolved in acetonitrile containing 0.1 % TFA. A lyophilized equivalent obtained by reverse phase chromatography corresponding to 7.5 µL plasma or 1000 to 5000 of in vitro cultured cells or 80 µg of tumour tissue or 30 µL cell culture supernatant spotted onto the mass spectrometric carrier plate was used to measure the peptides and/or proteins. The fractionated, lyophilized sample was dissolved in 15 µL of a matrix solution. This matrix solution contained, for example, 10 g/L α-cyano-4-hydroxycinnamic acid and 10 g/L L(-)fucose dissolved in a solvent mixture consisting of acetonitrile, water, trifluoroacetic acid (TFA) and acetone in a ratio of 49:49:1:1 by volume (v/v). 0.3 µL of this sample solution was transferred to a MALDI carrier plate, and the dried sample was analyzed in a Voyager-DE STR MALDI mass spectrometer from PerSeptive Biosystems. The measurement took place in linear mode with delayed extraction™. A MALDI-TOF mass spectrometer can be employed to quantify peptides such as, for example, the peptides of the invention if these peptides are present in a concentration which is within the dynamic measurement range of the mass spectrometer, thus avoiding detector saturation. The ability of MALDI mass spectrometry to quantitate individual peptide and/or protein signals was confirmed by adding known concentrations of 50 to 800 pmol of peptides, such as neurotensin, into complex biological samples such as plasma samples. Subsequently these samples were separated by reversed phase chromatography and those fractions containing the added peptides were analyzed by mass spectrometry as described in the examples. There is a the linear correlation between the MALDI mass spectrometry signal intensity of for example the neurotensin peptide and the amount of the neurotensin peptide added to the plasma sample, indicating that MALDI mass spectrometry is suitable to quantitatively determine peptides in complex samples such as plasma (figure 1).

### Example 10: Peptide identification

Detection of differentially expressed peptides was achieved by calculation of subtractive peptide display maps and correlation analysis. Selected peptides, which appeared in larger quantities in peptide display maps of plasma from mice with a tumour compared to plasma of tumour free mice, or which appeared in HTC-116 colon cancer tumours grown in mice, or which appeared in cell extracts of in vitro cultured HTC-116 colon cancer cells, or which peptides were present in different quantities in primary breast cancer specimens from estradiol-negative as compared to estradiol-positive breast cancer samples were analyzed by ESI-qTOF sequencing (PE Applied Biosystems, Framingham, USA). Peptide ions were selected in the mass spectrometer on the basis of their specific m/z (mass/charge) values in a manner known to the skilled worker. These selected ions were then fragmented by supplying collision energy (20 - 40 eV) with an collision gas, e.g. helium or nitrogen, and the resulting fragments of the peptides or proteins were detected in the mass spectrometer in an integrated analysis unit, and corresponding m/z values were determined (principle of tandem mass spectrometry). The fragmentation behaviour of peptides enables unambiguous identification of the peptides. The mass-spectrometric analysis for example can be done for example by use of a Quadrupol-TOF (time of flight) sequencing (QStar-Pulsar model) or by ESI-qTOF sequencing (both from PE Applied Biosystems, Framingham, USA). The resulting peptide fragment spectra were detected using nanoSpray in the product ion scan mode (spray voltage 950 V, collision energy 20-40 eV). Up to 200 scans per sample were accumulated. Charge state deconvolution was performed using the Bayesian reconstruct tool of the BioAnalyst program package (PE Applied Biosystems), deisotoping was achieved by means of the Voyager 5.1 software (PE Applied Biosystems). The mass spectra were saved in a MASCOT generic file format, and submitted to the MASCOT19 database search engine (Matrix Science, London, UK). Searched databases were SwissProt (Version 39.6, www.expasy.ch) and MSDB (Version 010721, EBI, Europe). In addition, this peptide sequencing method allows the identification of amino acid modifications such as phosphorylation or acetylation, that are often involved in the regulation of the biological activity of proteins and peptides.

MALDI-PSD-MS measurements of single HPLC-fractions of tissue specimen were also performed using a Voyager ToF mass spectrometer (Applied Biosystems, Framingham, MA, USA).

### Example 11: Data analysis

Subsequently to fractionation as described in example 7 or 8, each fraction was individually analyzed by MALDI mass spectrometry as described in example 9 resulting in 96 mass spectra for each sample. These 96 mass spectra are electronically combined to a so called peptide display. The x-axis of these peptide displays depicts the molecular mass, the y-axis depicts the fraction number and the grey scale colour intensity represents the mass spectrometric signal intensity. The data of peptide displays may be pre-processed by adjusting for background noise and outliers may be removed from the analysis. Differences between peptide displays are calculated by subtracting peptide displays from each other electronically and/or by correlation analysis of signals. For correlation analysis every signal above a certain threshold signal intensity was tested for correlation to any other signal above the same certain threshold of signal intensity. Detection of different (relative) concentrations of peptides and/or proteins was done by comparison of the mass spectrometric data (signal intensities) from samples to be compared, namely SCID mouse plasma from mice with the human colon carcinoma cell line HCT-116 implanted or without an implanted cell line or primary human breast cancer tissue extracts prepared from estradiol receptor negative breast cancer cells as compared to tissue extracts from estradiol receptor positive breast cancer cells. In addition peptide displays were generated from in vitro cultured cells of the colon carcinoma cell line HCT-116 and from HCT-116 tumour tissue isolated from SCID mice. Peptide displays gave about 1000 to 2300 non-redundant peptide and/or protein signals. Various peptide and/or protein signals were identified in plasma of SCID mice which clearly originated from the xenograft (the implanted, human HCT-116 colon tumour cell line). These peptides and/or proteins were detected in the plasma of the xenograft-transplanted SCID mice but not in the control mice injected with PBS instead of tumour cells. In addition some of these various peptides were also identified in vitro cultured HCT-116 cells and in HCT-116 tumour tissue isolated form SCID mice. Comparison of estradiol positive and negative primary breast cancer tissues also gave some differences in peptide signals. Both experimental setups resulted in the identification of certain peptides originating from the N-terminus of prothymosin alpha (PTA) and some of these PTA peptides were identified in both systems.

## Claims

1. A method for the stratification of an individual for the treatment of cancer to determine whether the cancer cells of said cancer will be susceptible to an anti-endocrine therapy comprising the steps of:
d) determining the amount of prothymosin-alpha (PTA) or a fragment thereof in a sample of an individual;
e) comparing the result of a) with the result determined using a negative control sample or with an already know reference value; and
f) determining the susceptibility of the cancer cells to an anti-endocrine therapy.

2. The method according to claim 1, wherein said amount of said PTA or fragment thereof is altered in said cancer cells being not susceptible to an anti-endocrine therapy in comparison to said negative control or said reference value.

3. The method according to claim 1 or 2, wherein said cancer cells not susceptible to an anti-endocrine therapy have an impaired steroid signalling pathway.

4. The method according to any one of claims 1 to 3, wherein said fragment of PTA is a peptide, which can be generated by action of the protease legumain.

5. The method according to any one of claims 1 to 4, wherein said cancer cells are selected from the group consisting of breast cancer cells, colon cancer cells, central nervous system cancer cells, prostate cancer cells and ovarian cancer cells.

6. The method according to any one of claims 1 to 5, wherein said sample is selected from the group consisting of whole blood, plasma, serum, lymph, nipple aspirate fluid, milk, urine and stool.

7. The method according to any one of the preceding claims, wherein the anti-endocrine therapy is an anti-estradiol therapy.

8. The method according to any one of the preceding claims, wherein said PTA or fragment of PTA is determined by a method selected form the group consisting of ELISA, RIA, western blot, protein chip assays, mass spectrometry, immune histology, flow cytometry or by molecular biologic methods.

9. Use of a method according to any one of the preceding claims for stratification of individuals treated with an anti-endocrine to identify individuals with cancer cells, which acquired a decreased susceptibility or a resistance to said therapy.

10. Use of a method according to any one of the preceding claims for stratification of individuals treated with a substance selected from the group consisting of anti-estrogenes, estrogene-modulating substances, aromatase inhibitors, Tamoxifen, Toremifen, Raloxifen, Exemestan, Letrozol and Anostrozol to identify individuals with cancer cells, which acquired a decreased susceptibility or a resistance to said substance.

11. Use of a method according to any one of the preceding claims in addition to the determination of
a) an endocrine receptor in a sample of an individual, and/or
b) an estradiol receptor in a sample of an individual, and/or
c) a progesterone receptor in a sample of an individual.

12. A peptide which is a fragment of PTA and/or a derivative and/or a polymorphic form thereof, which peptide is present in an altered amount in a sample of an individual diseased with cancer cells having a decreased susceptibility or a resistance to an endocrine therapy as compared to an individual diseased with cancer cells being susceptible to an anti-endocrine therapy with the proviso that said peptide is not Seq.-ID 11 to 14.

13. The peptide according to claim 12, wherein said peptide is
a) a peptide having a sequence according to Seq.-ID 1 to 10; and/or
b) a polymorphic form of a peptide according to a); and/or
c) a derivative of a peptide according to a) or b).

14. Binding agents specifically binding to a neoepitope of a peptide according to Seq.-ID 1 to 10, which do not bind a peptide according to Seq.-ID 11 to 14.

15. Binding agents according to claim 14 which are antibodies, fragments or derivatives thereof.

16. Nucleic acid selected form the group consisting of
a) a nucleic acid sequence encoding a peptide according to any one of claim 12 or 13;
b) a nucleic acid sequence which is complementary to the nucleic acid sequence under a);
c) a nucleic acid sequence, which hybridizes under stringent conditions to a nucleic acid under a) and/or b) and which at maximum has a nucleic acid sequence length which is shorter than the nucleic acid sequence coding for Seq.-ID 14 and with the proviso that said nucleic acid sequence does not encode TA1 or TA11 according to Seq.-ID 11. or 12 or 13;
d) a nucleic acid sequence, which is degenerated with respect to said nucleic acid sequence under any one of a) to c);
e) a derivative and/or fragment of a nucleic acid sequence under any one of a) to d) with the proviso that said nucleic acid sequence does not encode TA1 or TA11 according to Seq.-ID 11 or 12;
f) a nucleic acid sequence according to a), b), c), d) or e) further comprising at least one non-PTA nucleic acid sequence; and/or
g) a nucleic acid sequence according to f), wherein said at least one non-PTA nucleic acid sequence is a sequence for labelling of the nucleic acid sequence.

17. The nucleic acid according to claim 16 further comprising a label, which label is not a nucleic acid sequence.

18. A vector comprising a nucleic acid according to claim 16.

19. A host cell comprising a nucleic acid according to any one of claims 16 or 17 or a vector according to claim 18.

20. A testkit comprising
a) a peptide according to claim 12 or 13; and/or
b) a binding agent according to claim 14 or 15; and/or
c) a nucleic acid according to claim 16 or 17; and/or
d) a vector according to claim 18; and/or
e) a host cell according to claim 19, and
instructions how to use the test kit for determining the presence, absence, prognosis or relapse of a disorder.

21. A testkit comprising
a) a peptide according to claim 12 or 13; and/or
b) a peptide which is PTA or a fragment and/or a derivative and/or a polymorphic form thereof; and/or
c) a peptide according to b) which in addition comprises at least one non-PTA amino acid; and/or
d) a binding agent according to claim 14 or 15; and/or
e) a binding agent specific for PTA or specific for a fragment or derivative or polymorphic form thereof; and/or
f) a nucleic acid according to claim 16 or 17; and/or
g) a nucleic acid which is coding for PTA and/or a fragment and/or derivative and/or a polymorphic form thereof; and/or
h) a nucleic acid sequence which is complementary to or which hybridizes under stringent conditions to a nucleic acid according to f) or g); and/or
i) a nucleic acid according to f) to h) which in addition comprises at least one non-PTA nuleotide; and/or
j) a vector according to claim 18; and/or
k) a host cell according to claim 19, and
instructions how to use said testkit for a method according to any one of claims 1 to 8 or instructions how to use said testkit for a use according to claim 9 to 11.

22. A pharmaceutical composition comprising
a) a peptide according to claim 12 or 13; and/or
b) a binding agent according to claim 14 or 15; and/or
c) a nucleic acid according to claim 16 or 17; and/or
d) a vector according to claim 18; and/or
e) a host cell according to claim 19.

23. A pharmaceutical composition for treatment and/or prophylaxis and/or diagnosis of a disease selected from the group consisting of breast cancer, colon cancer, central nervous system cancer, prostate cancer and ovarian cancer, comprising
a) a peptide according to claim 12 or 13; and/or
b) a peptide which is PTA or a fragment of PTA and/or a derivative and/or a polymorphic form thereof, with the proviso that Seq.-IDs 11 to 13 are not included; and/or
c) a peptide according to b) which in addition comprises at least one non-PTA amino acid, and/or
d) a binding agent according to claim 14 or 15; and/or
e) a binding agent specific for PTA or specific for a fragment or derivative or polymorphic form thereof; and/or
f) a nucleic acid according to claim 16 or 17; and/or
g) a nucleic acid coding for PTA and/or a fragment and/or derivative and/or a polymorphic form thereof; and/or
h) a nucleic acid sequence which is complementary to or which hybridizes under stringent conditions to a nucleic acid according to f) or g); and/or
i) a nucleic acid according to f) to h) which in addition comprises at least one non-PTA nuleotide; and/or
j) a vector according to claim 18; and/or
k) a host cell according to claim 19; and/or
l) a host cell comprising a nucleic acid according to f) to i), or a vector according to j).

24. Use of
a) a peptide according to any one of claims 12 to 13; and/or
b) a binding agent according to claim 14 or 15; and/or
c) a nucleic acid according to claim 16 or 17; and/or
d) a vector according to claim 18; and/or
e) a host cell according to claim 19
for the manufacture of a testkit according to 20 or 21 and/or a pharmaceutical composition according to claim 22 or 23.

25. Use of
a) a peptide according to claim 12 or 13; and/or
b) a peptide which is PTA or a fragment of PTA and/or a derivative and/or a polymorphic form thereof, with the proviso that Seq.-IDs 11 to 13 are not included; and/or
c) a peptide according to b) which in addition comprises at least one non-PTA amino acid, and/or
d) a binding agent according to claim 14 or 15; and/or
e) a binding agent specific for PTA or specific for a fragment or derivative or polymorphic form thereof; and/or
f) a nucleic acid according to claim 16 or 17; and/or
g) a nucleic acid coding for PTA and/or a fragment and/or derivative and/or a polymorphic form thereof; and/or
h) a nucleic acid sequence which is complementary to or which hybridizes under stringent conditions to a nucleic acid according to f) or g); and/or
i) a nucleic acid according to f) to h) which in addition comprises at least one non-PTA nuleotide; and/or
j) a vector according to claim 18; and/or
k) a host cell according to claim 19; and/or
l) a host cell comprising a nucleic acid according to f) to i), or a vector according to j).
for the manufacture of a testkit and/or a pharmaceutical composition for treatment and/or prophylaxis and/or diagnosis of a disease selected from the group consisting of breast cancer, colon cancer, central nervous system cancer, prostate cancer and ovarian cancer.
